# FASCICULE DE BREVET EUROPEEN

(11) **EP 1 588 687 B1**
(45) Date de publication et mention de la délivrance du brevet: **17.07.2013**
(21) Numéro de dépôt: 05300267.1
(22) Date de dépôt: 08.04.2005
(51) Int. Cl.: A61K 8/25, A61K 8/29, A61Q 1/02, A61Q 1/06, A61Q 1/10, A61Q 3/02, A61K 8/11, A61K 8/19, A61K 8/46

(54) **Composition destinée à être appliquée sur la peau, les lèvres et/ou les phanères**
Zusammensetzung zur Applikation auf die Haut, die Lippen und/oder die Nägel
Composition for use on the skin, lips and/or nails

(30) Priorité: 08.04.2004 FR 0450712
(43) Date de publication de la demande: 26.10.2005
(73) Titulaire: L'Oréal, 75008 Paris (FR)
(72) Inventeur: Dumousseaux, Christophe, Tokyo (JP)
(74) Mandataire: Tanty, François

(56) Documents cités:
- EP-A- 1 184 426
- EP-A- 1 217 046
- EP-A- 1 382 323
- FR-A- 2 594 130
- US-A- 5 356 617
- US-A1- 2002 064 509
- US-B1- 6 428 773

## Description

La présente invention concerne les compositions cosmétiques destinées à être appliquées sur la peau, y compris les muqueuses, notamment les lèvres, et les phanères, notamment les ongles, cils, sourcils et cheveux.

Il est connu de préparer des compositions comportant un agent de coloration apte à réfléchir la lumière et/ou à modifier la perception visuelle de la composition cosmétique. En association avec cet agent de coloration, il est connu d'incorporer dans la composition des pigments de nature organique ou inorganique sous forme pure, ce qui présente l'inconvénient de rendre la composition insuffisamment transparente pour bénéficier au mieux des effets optiques produits par l'agent de coloration précité.

Le brevet US 6 428 773 divulgue une composition cosmétique comportant un pigment interférentiel ayant une structure multicouche particulière et éventuellement un pigment interférentiel conventionnel.

Les demandes de brevet européen EP 1 217 046 et EP 1 184 426 divulguent des peintures ou résines et non des compositions destinées à être appliquées sur la peau, les lèvres ou les phanères.

La demande EP 1 382 323 ne divulgue pas un pigment composite non interférentiel comportant un noyau inorganique au moins partiellement recouvert par une matière colorante organique.

Le brevet US 5 356 617 ne recherche par l'obtention d'effets optiques spécifiques par association de matières colorantes.

La demande de brevet français FR 2 594 130 divulgue un rouge à lèvres réalisé avec des pigments composites de taille relativement grande (10 µm) et ayant un noyau organique (méthlylméthacrylate). Aucun effet optique spécifique n'est recherché.

Les demandes de brevet français FR 2 845 277 et FR 2 848 821 constituent un état de la technique opposable seulement au titre de la nouveauté, selon l'article 54(3) CBE.

La demande FR 2 845 277 ne recherche pas l'obtention d'un effet optique spécifique par association de plusieurs agents de coloration et la demande FR 2 848 821 ne divulgue pas de pigment composite non interférentiel comportant un coeur inorganique recouvert au moins partiellement d'une matière colorante organique.

Il existe un besoin pour disposer d'une composition cosmétique présentant une coloration satisfaisante et comportant un agent de coloration apte à produire un effet optique spécifique suffisamment visible.

L'invention vise notamment à répondre à ce besoin.

Elle y parvient grâce à une composition destinée à être appliquée sur la peau, les lèvres et/ou les phanères, comportant :
- entre environ 0,1 % et 20 % environ en poids, par rapport au poids total de la composition, d'au moins un premier agent de coloration dans une quantité suffisante pour colorer la composition, comportant des particules d'au moins un pigment composite non interférentiel, ces particules comportant :
- un noyau inorganique,
- un enrobage au moins partiel d'au moins une matière colorante organique,
   cette composition comportant en outre :
- entre environ 0,1 % et environ 50 % en poids, par rapport au poids total de la composition, d'au moins un deuxième agent de coloration dans une quantité suffisante pour produire dans la composition un effet optique spécifique perceptible à l'oeil nu,
le deuxième agent de coloration comportant des particules réfléchissantes, des nacres et/ou un agent de coloration goniochromatique.

L'effet optique spécifique peut être un effet de brillance ponctuelle, un effet goniochromatique, un effet tacheté, ou tout autre effet optique recherché.

Un effet de brillance ponctuelle peut se traduire par exemple par des points de surbrillance au sein de la composition, contrastant avec la couleur environnante. Ces points de surbrillance peuvent être dus à des particules réfléchissantes par exemple.

Un effet goniochromatique se traduit par une variation de la couleur en fonction de l'angle d'observation.

Un effet tacheté peut se traduire par la présence de taches de couleur visibles à l'oeil au sein de la composition, ces taches étant dues par exemple à des paillettes ou fibres.

Grâce à l'invention, on peut obtenir des compositions à la fois colorées et présentant un effet optique visible à l'oeil nu, dû à la présence du deuxième agent de coloration.

Le premier agent de coloration présent dans la composition apporte quant à lui de la couleur tout en conservant à la composition la transparence ou translucidité requise pour que l'effet optique apporté par le deuxième agent de coloration reste visible à l'oeil nu.

La teneur en premier agent de coloration dans la composition peut être comprise entre environ 0,1 % et environ 20 % en poids par rapport au poids total de la composition, voire entre environ 0,1 % et environ 10 %, par exemple entre 0,5 % et 5 % en poids par rapport au poids total de la composition.

La teneur en deuxième agent de coloration dans la composition peut être comprise entre 0,1 % environ et 50 % environ en poids par rapport au poids total de la composition, voire entre 0,5 % environ et 40 % environ, par exemple entre 1 % environ et 20 % environ en poids par rapport au poids total de la composition.

Le rapport en poids du deuxième agent de coloration au premier agent de coloration peut être compris entre environ 0,1 et environ 50, voire entre 0,5 environ et 30 environ, par exemple entre environ 1 et environ 10.

Dans des exemples de mise en oeuvre de l'invention, la quantité du premier agent de coloration peut être inférieure à la quantité du second agent de coloration.

Le pigment composite peut être différent d'un pigment interférentiel tel que décrit par exemple dans le brevet US 6 428 773. Un pigment interférentiel comporte par exemple plusieurs couches d'épaisseurs constantes de matériaux sélectionnés pour pouvoir produire des interférences optiques.

La saturation C* du pigment composite peut être supérieure ou égale à 30, mesurée selon le protocole ci-dessous.

### Protocole de mesure de la saturation du pigment composite :

Les valeurs a* et b* dans l'espace CIE L*a*b* du pigment composite sont mesurées comme suit :
Le pigment composite pur est compacté dans une coupelle rectangulaire ayant pour dimensions 2 x 1,5 cm et une profondeur de 3 mm, en appliquant une pression de 100 bars.

Les valeurs a* et b* du pigment compacté sont mesurées avec un spectrophotomètre MINOLTA 3700d, en mode spéculaire exclu, sous illuminant D65, ouverture moyenne. La saturation est donnée par C* = (a*² + b*²)^{1/2}.

Une teinte appropriée peut être obtenue de diverses façons, par exemple par le mélange de pigments composites selon l'invention, ces pigments ayant des couleurs différentes et/ou par la présence de plusieurs matières colorantes organiques dans l'enrobage des noyaux du ou des pigments composites, ces matières colorantes organiques étant par exemple mélangées ou présentes dans des strates respectives de l'enrobage.

Par « un enrobage au moins partiel », on entend au sens de la présente invention, un enrobage de tout ou partie du noyau inorganique.

La composition selon l'invention peut comporter un milieu physiologiquement acceptable.

Par « milieu physiologiquement acceptable », on désigne un milieu non toxique et susceptible d'être appliqué sur la peau, les lèvres ou les phanères d'êtres humains notamment un milieu cosmétique. Le milieu physiologiquement acceptable sera adapté à la nature du support sur lequel doit être appliquée la composition, ainsi qu'à la forme sous laquelle la composition est destinée à être conditionnée, notamment solide ou fluide à température ambiante et sous pression atmosphérique.

Par « composition cosmétique », on désigne une composition telle que définie dans la Directive 93/35/CEE du Conseil du 14 juin 1993.

### PIGMENT COMPOSITE DUPREMIER AGENT DE COLORATION Structure

Un pigment composite du premier agent de coloration selon l'invention peut être composé notamment de particules comportant :
- un noyau inorganique,
- au moins un enrobage au moins partiel de matière colorante organique.

Au moins un liant peut avantageusement contribuer à la fixation de la matière colorante organique sur le noyau inorganique.

Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5.

Un pigment composite selon l'invention peut présenter par exemple une surface spécifique comprise entre 1 et 1000 m²/g, notamment entre 10 et 600 m²/g environ, et en particulier entre 20 et 400 m²/g environ. La surface spécifique est la valeur mesurée par la méthode BET.

La composition peut comporter uniquement un ou plusieurs pigments composites tels que définis plus haut ou en variante comporter un ou plusieurs pigments composites autres ainsi que des pigments présentant une structure non composite, notamment des pigments minéraux, des laques ou des pigments organiques. La composition peut notamment être dépourvue de particules de TiO₂ non enrobées.

Le pigment composite selon l'invention est différent du deuxième agent de coloration qui, lui, est apte à produire dans la composition un effet optique spécifique. Le pigment composite est différent d'un pigment interférentiel.

### Noyau inorganique

Le noyau inorganique peut être de toute forme convenant à la fixation de particules de matière colorante organique, par exemple sphérique, globulaire, granulaire, polyédrique, aciculaire, fusiforme, aplatie en forme de flocon, de grain de riz, d'écaille, ainsi qu'une combinaison de ces formes, cette liste n'étant pas limitative.

De préférence, le rapport de la plus grande dimension du noyau à sa plus petite dimension est compris entre 1 et 50.

Le noyau inorganique peut présenter une taille moyenne comprise entre environ 1 nm et environ 100 nm, voire entre environ 5 nm et environ 75 nm, par exemple entre environ 10 nm et environ 50 nm.

Par « taille moyenne », on désigne la dimension donnée par la distribution granulométrique statistique à la moitié de la population, dite D50. La taille moyenne peut être une taille moyenne en nombre déterminée par analyse d'image (microscopie électronique).

Le noyau inorganique peut présenter un indice de réfraction supérieur ou égal à 2, voire supérieur ou égal à 2,1, par exemple supérieur ou égal à 2,2.

Le noyau inorganique peut être réalisé dans un matériau choisi dans la liste non limitative comprenant les sels métalliques et oxydes métalliques, notamment les oxydes de titane, de zirconium, de cérium, de zinc, de fer, de bleu ferrique, d'aluminium et de chrome, les alumines, les verres, les céramiques, le graphite, les silices, les silicates, notamment les aluminosilicates et les borosilicates, le mica synthétique, et leurs mélanges.

Les oxydes de titane, notamment TiO₂, de fer, notamment Fe₂O₃, de cérium, de zinc et d'aluminium, les silices et les silicates, notamment les aluminosilicates et les borosilicates conviennent tout particulièrement.

Le noyau inorganique peut présenter une surface spécifique, mesurée par la méthode BET, comprise par exemple entre environ 1 m²/g et environ 1000 m²/g, mieux entre environ 10 m²/g et environ 600 m²/g, par exemple entre environ 20 m²/g et environ 400 m²/g.

Le noyau inorganique peut être coloré, le cas échéant.

La proportion massique du noyau peut excéder 50 % par rapport au poids total du pigment composite, par exemple aller de 50 % à 70 %, par exemple de 60 % à 70 %.

### Matière colorante organique

La matière colorante organique peut comporter par exemple au moins un pigment organique, par exemple au moins une laque organique ou autre pigment organique.

La matière colorante organique peut être choisie par exemple parmi les composés particulaires insolubles dans le milieu physiologiquement acceptable de la composition.

La matière colorante organique peut comporter par exemple des pigments, notamment des laques organiques ou autres pigments, qui peuvent être choisis parmi les composés ci-dessous et leurs mélanges :
- le carmin de cochenille,
- les pigments organiques de colorants azoïques, anthraquinoniques, indigoïdes, xanthéniques, pyréniques, quinoliniques, de triphénylméthane, de fluorane,
- les laques organiques ou sels organiques insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Parmi les pigments organiques, on peut notamment citer ceux connus sous les dénominations suivantes : D&C Blue n° 4, D&C Brown n° 1, D&C Green n° 5, D&C Green n° 6, D&C Orange n° 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

La matière colorante organique peut comporter une laque organique supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les composés chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association », dont le contenu est incorporé dans la présente demande par référence.

La proportion massique de matière colorante organique peut être comprise entre environ 10 parts et environ 500 parts en poids pour 100 parts du noyau inorganique, voire entre environ 20 parts et environ 250 parts en poids, par exemple entre environ 40 parts et environ 125 parts en poids pour 100 parts du noyau inorganique.

La proportion de la matière colorante organique peut excéder 30 % par rapport au poids total du pigment composite, par exemple aller de 30 à 50 %, par exemple de 30 à 40%.

### Liant

Le liant peut être de tout type dès lors qu'il permet à la matière colorante organique d'adhérer à la surface du noyau inorganique. Le liant peut être organique

Le liant peut notamment être choisi parmi une liste non limitative comprenant les composés siliconés, les composés polymériques, oligomériques ou similaires, et en particulier parmi les organosilanes, les organosilanes fluoroalkylés et les polysiloxanes, par exemple le polyméthylhydrogénosiloxane, ainsi que divers agents couplants, tels que des agents couplants à base de silanes, de titanates, d'aluminates, de zirconates et leurs mélanges.

Le composé siliconé peut être choisi parmi une liste non limitative comprenant notamment :
- les organosilanes (1) obtenus à partir d'alkoxysilanes,
- les polysiloxanes (2) modifiés ou non choisis parmi une liste non limitative comprenant :
   - les polysiloxanes modifiés (2A) comprenant au moins un radical choisi parmi, notamment, les polyéthers, les polyesters et les composés époxy (ils seront appelés « polysiloxanes modifiés »),
   - les polysiloxanes (2B) portant sur un atome de silicium situé à l'extrémité du polymère, au moins un groupe choisi parmi une liste non limitative comprenant les acides carboxyliques, les alcools ou les groupes hydroxy, et
- les composés organosilanes fluoroalkylés (3) obtenus à partir de fluoroalkylsilanes.

Les composés organosilanes (1) peuvent être obtenus à partir de composés alkoxysilanes représentés par la formule (I) :

R¹ ₐ Si X₄₋ₐ (I)

dans laquelle :
- R¹ représente C₆H₅-, (CH₃)₂ CH-CH₂- ou un radical de type C_{b} H_{2b+1}- (où b varie de 1 à 18),
- X représente CH₃O- ou C₂H₅O-, et
- a varie de 0 à 3.

Des exemples spécifiques de composés alkoxysilanes peuvent inclure les alkoxysilanes choisis parmi : le méthyltriéthoxysilane, le diméthyldiéthoxysilane, le phényltriéthyoxysilane, le diphényldiéthoxysilane, le méthyltriméthoxysilane, le diméthyldiméthoxysilane, le phényltriméthoxysilane, le diphényldiméthoxysilane, l'isobutyltriméthoxysilane, le décyltriméthoxysilane et similaires, en particulier parmi le méthyltriéthoxysilane, le phényltriéthoxysilane, le méthyltriméthoxysilane, le diméthyldiméthoxysilane, l'isobutyltriméthoxysilane, et encore mieux le méthyltriéthoxysilane, le méthyltriméthoxysilane, le phényltriéthoxysilane.

Les polysiloxanes (2) peuvent notamment répondre à la formule (II) : 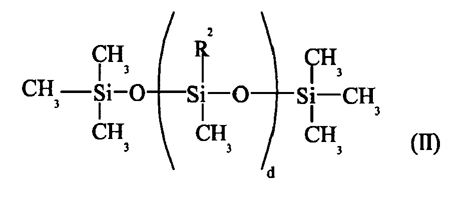 dans laquelle R² représente H- ou CH₃- et d varie de 15 à 450.

Parmi ces polysiloxanes, ceux pour lesquels R² représente H sont préférés.

Les polysiloxanes modifiés (2A) peuvent notamment répondre aux formules suivantes :
- (a¹) polysiloxanes modifiés portant des polyéthers, représentés par la formule (III) 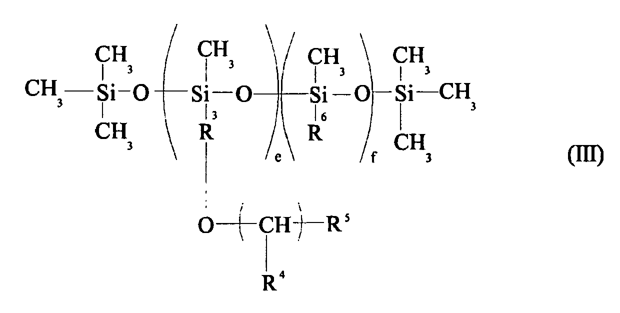 dans laquelle R³ représente -(CH₂)ₕ- ; R⁴ représente -(CH₂)ᵢ- CH₃ ; R⁵ représente -OH, - COOH, -CH = CH₂, -C (CH₃) = CH₂ ou -(CH₂)ⱼ- CH₃ ; R⁶ représente -(CH2)ₖ- CH₃ ; g et h variant indépendamment de 1 à 15 ; j et k variant indépendamment de 0 à 15 ; e variant de 1 à 50 et f variant de 1 à 300,
- (a²) polysiloxanes modifiés portant des polyesters, représentés par la formule (IV) : 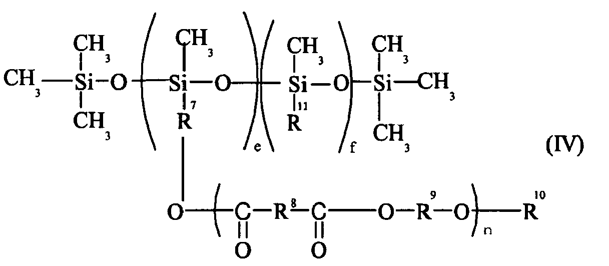 dans laquelle R⁷, R⁸ et R⁹ représentent indépendamment -(CH₂)_{q}- ; R¹⁰ représente -OH ; -COOH, -CH = CH₂, -C(CH₃) = CH₂ ou -(CH₂)ᵣ- CH₃ ; R¹¹ représente -(CH₂)ₛ- CH₃ ; n et q variant indépendamment de 1 à 15, r et s variant indépendamment de 0 à 15 ; e variant de 1 à 50 et f variant de 1 à 300,
- (a³) polysiloxanes modifiés portant des radicaux époxy représentés par la formule (V) : 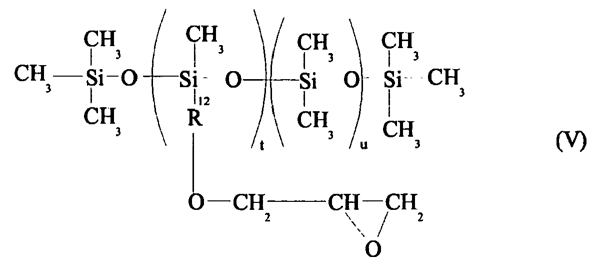
dans laquelle R¹² représente -(CH₂)ᵥ- ; v variant de 1 à 15 ; t variant de 1 à 50 et u variant de 1 à 300 ; ou leurs mélanges.
Parmi les polysiloxanes modifiés (2A), les polysiloxanes modifiés portant des polyéthers de formule (III) sont préférés.
Les polysiloxanes modifiés sur la partie terminale (2B) peuvent répondre à la formule (VI) : 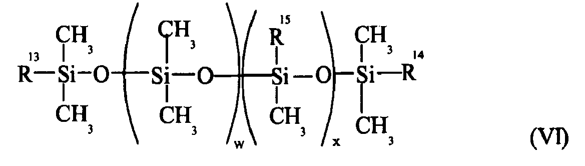 dans laquelle R¹³ et R¹⁴ peuvent représenter -OH, R¹⁶-OH ou R¹⁷-COOH, indépendamment l'un de l'autre ; R¹⁵ représente -CH₃ ou -C₆H₅ ; R¹⁶ et R¹⁷ représentent - (CH₂)_{y}- ; y variant de 1 à 15 ; w variant de 1 à 200 et x variant de 0 à 100.

Parmi ces polysiloxanes modifiés sur au moins une extrémité, ceux portant au moins radical (R¹⁶ et/ou R¹⁷) portant un groupement acide carboxylique sur au moins un atome de silicium terminal sont encore préférés.

Les composés organosilanes fluoroalkylés (3) peuvent être obtenus à partir de fluoroalkyles silanes représentés par la formule (VII) :

CF₃(CF₂)_{z}CH₂CH₂ (R¹⁸)ₐ SiX₄₋ₐ (VII)

dans laquelle :
- R¹⁸ représente CH₃-, C₂H₅-, CH₃O- ou C₂H₅O-,
- X représente CH₃O- ou C₂H₅O-,
- Z varie de 0 à 15 et a varie de 0 à 3.

Les fluoroalkylsilanes peuvent notamment être choisis dans une liste non limitative comprenant notamment le trifluoropropyltriméthoxysilane, le tridécafluorooctyltriméthoxysilane, l'heptadécafluorodécyltriméthoxysilane, l'heptadécafluorodécylméthyldiméthoxysilane, le trifluoropropyltriéthoxysilane, le tridécafluorooctyltriéthoxysilane, l'heptadécafluorodecyltriéthoxysilane, l'heptadécafluorodécylméthyldiéthoxysilane et similaires, en particulier le trifluoropropyltriméthoxysilane, le tridécafluorooctyltriméthoxysilane et l'heptadécafluorodécyltriméthoxysilane, et encore mieux le trifluoropropyl triméthoxysilane et le tridécafluorooctyltriméthoxysilane.

Les agents couplants à base de silane peuvent être choisis parmi une liste non limitative comprenant notamment le vinyltriméthoxysilane, le vinyltriéthoxysilane, γ -aminopropyl-triéthoxysilane, le γ-flycidoxypropyltriméthoxysilane, le γ -mercaptopropyltriméthoxysilane, le γ-méthacryloxypropyltriméthoxysilane, le N-β(aminoéthyl)-γ-aminopropyltriméthoxysilane, le γ -glycidoxypropylméthyldiméthoxysilane, le γ-chloropropyltriméthoxysilane et similaires.

Les agents couplants à base de titanate peuvent être choisis dans la liste comprenant le titanate d'isopropylstéaroyle, le titanate d'isopropyltris(dioctylpyrophosphate), le titanate d'isopropyltri(N-aminoéthylaminoéthyl), le titanate de tétraoctylbis(ditridécylphosphate), le titanate de tétra(2,2-diaryloxyméthyl-1-butyl)bis(ditridécyl)phosphate, le titanate de bis(dioctylpyrophosphate)oxyacétate, le titanate de bis(dioctylpyrophosphate)éthylène et leurs similaires.

Les agents couplants à base d'aluminate peuvent être choisis parmi les diisopropylate d'acétoalkoxyaluminium, le diisopropoxymonoéthylacétoacétate d'aluminium, le triéthylacétoacétate d'aluminium, le triacétylacétonate d'aluminium et leurs similaires.

Les agents couplants à base de zirconate peuvent être choisis dans une liste comprenant notamment le tétrakisacétylacétonate de zirconium, le dibutoxybisacétylacétonate de zirconium, le tétrakiséthylacétoacétate de zirconium, le tributoxymonoéthylacétoacétate de zirconium, le tributoxyacétylacétonate de zirconium et leurs similaires.

Les composés servant de liant peuvent notamment présenter une masse molaire pouvant varier entre 300 et 100 000.

Pour obtenir une couche recouvrant les noyaux inorganiques uniformément, le liant est de préférence dans un état liquide ou soluble dans l'eau ou dans différents solvants.

La quantité de liant peut varier de 0,01 à 15 %, notamment de 0,02 à 12,5 % et en particulier de 0,03 à 10% en poids (calculée par rapport à C ou Si) par rapport au poids des particules comprenant le noyau et le liant. Pour de plus amples détails sur la manière de calculer la quantité relative du liant, on pourra se reporter à la demande EP 1 184 426 A2. La quantité de liant est par exemple inférieure ou égale à 5 %, par exemple inférieure ou égale à 3 %, en poids relativement au poids total du pigment composite.

### Préparation du pigment composite

Le pigment composite peut être préparé par tout procédé approprié, par exemple un procédé méchano-chimique ou un précédé de précipitation en solution, avec dissolution de la matière colorante organique puis précipitation à la surface du noyau.

Un liant peut être utilisé ou non.

Un procédé comportant un mélange mécanique d'un pigment organique et du noyau inorganique est préféré.

Un liant peut être ajouté et mélangé au noyau inorganique avant l'introduction de la matière colorante organique.

Le pigment composite peut être réalisé par exemple par l'un des procédés décrits dans les demandes de brevet européen EP 1 184 426 et EP 1 217 046, avantageusement par le procédé décrit dans la demande EP 1 184 426.

Dans un exemple de mise en oeuvre, on commence par mélanger les particules destinées à constituer le noyau inorganique avec le liant.

De manière à ce que le liant adhère uniformément à la surface du noyau inorganique, il est préférable de passer ces particules au préalable dans un broyeur, de façon à les désagglomérer.

Les conditions de mélange et d'agitation sont choisies de manière à ce que le noyau soit uniformément recouvert de liant. Ces conditions peuvent être contrôlées pour que la charge linéaire soit comprise entre 19,6 et 19160 N/cm, en particulier entre 98 et 14170 N/cm et mieux entre 147 et 980 N/cm ; le temps de traitement est notamment compris entre 5 mn et 24 heures et mieux de 10 mn à 20 heures ; la vitesse de rotation peut être comprise entre 2 et 1000 trs/mn, en particulier entre 5 et 1000 trs/mn et mieux entre 10 et 800 trs/mn.

Après que le liant a recouvert le noyau inorganique, la matière colorante organique est ajoutée et mélangée avec agitation pour adhérer à la couche de liant.

Les méthodes d'addition peuvent être par exemple une addition par grosse quantité, en continu, ou par petite quantité.

Le mélange et l'agitation, que ce soit des noyaux inorganiques avec le liant ou de la matière colorante organique avec les noyaux inorganiques recouverts de liant, peut être effectué en utilisant un appareil pouvant appliquer une force tranchante spatulaire et/ou de compression au mélange de poudres. De tels appareillages sont par exemple des malaxeurs à roues, à lames et similaires. Les malaxeurs à roues conviennent tout particulièrement. Une liste d'appareils pouvant convenir est donnée dans la demande EP 1 184 426 A2.

Une autre méthode de fabrication d'un pigment composite est décrite dans le brevet JP 3286463, qui divulgue un procédé de précipitation en solution.

La matière colorante organique est dissoute dans l'éthanol, les noyaux inorganiques sont ensuite dispersés dans cette solution éthanolique.

Ensuite, on ajoute lentement sur ces mélanges une solution aqueuse alcaline de carbonate de sodium ou de potassium, puis en dernier, lentement, une solution éthanolique de chlorure de calcium, le tout sous agitation.

### DEUXIEME AGENT DE COLORATION

Le deuxième agent de coloration est apte à produire, dans la composition, un effet optique spécifique.

Le deuxième agent de coloration comporte des particules réfléchissantes, des nacres et/ou un agent de coloration goniochromatique, entre autres.

Quel qu'il soit, le deuxième agent de coloration peut comporter des particules présentant une forme de plaquettes ou une forme globulaire.

Le deuxième agent de coloration peut présenter une structure multicouche.

Dans ce cas, le deuxième agent de coloration peut comporter un substrat comportant au moins l'un d'un métal, d'un oxyde métallique et d'un polymère, cette liste n'étant pas limitative.

Le deuxième agent de coloration peut comporter au moins l'un d'un mica, d'un mica synthétique, d'un talc, d'une silice et d'une alumine.

Le deuxième agent de coloration peut comporter des particules à base de verre, des particules métallisées en surface, des particules comportant un métal choisi parmi l'aluminium, le bronze, le cuivre et leurs alliages.

### Particules réfléchissantes

Par « particules réfléchissantes », on désigne au sens de la présente invention des particules dont la taille, la structure, notamment l'épaisseur de la ou des couches qui la constituent et leur natures physique et chimique, et l'état de surface, leur permettent de réfléchir la lumière incidente. Cette réflexion peut, le cas échéant, posséder une intensité suffisante pour créer à la surface de la composition selon l'invention, lorsque cette dernière est appliquée sur le support à maquiller, des points de surbrillance visibles à l'oeil nu, c'est-à-dire des points plus lumineux qui contrastent avec leur environnement en semblant briller.

Le cas échéant, les particules réfléchissantes peuvent être destinées à troubler la perception visuelle de la courbure du support maquillé, en tendant à empêcher une focalisation visuelle durable, les points de surbrillance étant susceptibles d'apparaître ou de disparaître de manière aléatoire lorsque le support maquillé et l'observateur sont animés.

Les particules réfléchissantes peuvent être également sélectionnées de manière à ne pas altérer significativement l'effet de coloration généré par le premier agent de coloration qui lui est associé, voire de manière à optimiser cet effet en terme de rendu de couleur. Elles peuvent plus particulièrement posséder une couleur ou un reflet jaune, rose, rouge, bronze, orangé, brun, or et/ou cuivré.

Les particules réfléchissantes utilisées doivent être compatibles avec une utilisation en cosmétique et doivent pouvoir subsister dans le milieu physiologiquement acceptable, et notamment ne pas se dissoudre, ou en tout cas ne pas se dissoudre entièrement, dans celui-ci.

Ces particules solides peuvent présenter des formes variées. Ces particules peuvent être notamment en forme de plaquettes ou globulaires, en particulier sphériques.

Les particules présentant une surface extérieure sensiblement plane conviennent également, car elles peuvent plus facilement donner naissance, si leur taille, leur structure et leur état de surface le permettent, à une réflexion spéculaire intense. On parle d'effet miroir.

Pour de telles particules notamment, c'est essentiellement la lumière renvoyée par réflexion dans une direction faisant, avec la normale à la surface réfléchissante, le même angle que celui que fait la lumière incidente avec cette normale, qui permet à ces particules d'apparaître comme des points de surbrillance, et non la lumière diffusée dans les autres directions.

Il peut être souhaitable que les particules réfléchissantes soient non diffusantes et non mates.

Il peut être souhaitable également que les particules réfléchissantes n'altèrent pas de manière sensible la coloration de la composition cosmétique.

A cet égard, les particules réfléchissantes qui permettent une réflexion métallique de la lumière incidente conviennent tout particulièrement. C'est le cas notamment lorsque les particules réfléchissantes permettent quelle que soit leur forme, une réflexion sur une couche d'un métal, par exemple de l'argent. De telles particules s'avèrent relativement neutres vis-à-vis de la couleur de la composition.

Des particules réfléchissantes utilisables dans l'invention, à reflet métallique ou blanc, peuvent par exemple réfléchir la lumière dans toutes les composantes du visible sans absorber de manière significative une ou plusieurs longueurs d'ondes. La réflectance spectrale de ces particules réfléchissantes peut par exemple être supérieure à 70 % dans l'intervalle 400-700 nm, et mieux d'au moins 80 %, voire 90 % ou encore 95 %.

La lumière réfléchie par les particules réfléchissantes peut être non iridescente, notamment dans le cas d'un reflet métallique.

Les particules réfléchissantes quelle que soit leur forme, peuvent présenter une structure multicouche ou non et, dans le cas d'une structure multicouche, par exemple au moins une couche d'épaisseur uniforme, notamment d'un matériau réfléchissant.

Lorsque les particules réfléchissantes ne présentent pas de structure multicouche, elles peuvent être composées par exemple d'oxydes métalliques, par exemple des oxydes de titane ou de fer obtenus par synthèse, de manière à présenter une surface sensiblement plane ayant un état de surface, par exemple non mat et non diffusant, permettant une réflexion spéculaire de la lumière suffisante pour obtenir des points de surbrillance au sein de la composition cosmétique.

Lorsque les particules réfléchissantes présentent une structure multicouche, celles-ci peuvent par exemple comporter un substrat naturel ou synthétique, notamment un substrat synthétique au moins partiellement enrobé par au moins une couche d'un matériau réfléchissant notamment d'au moins un métal ou composé métallique. Le substrat peut être monomatière, multimatériau, organique et/ou inorganique, plein ou creux.

Quelle que soit la forme des particules réfléchissantes, le substrat peut, lorsqu'il est synthétique, être réalisé avec une forme favorisant la formation d'une surface réfléchissante après revêtement, notamment après dépôt d'une couche de matériau réfléchissant. Le substrat peut, par exemple, présenter une surface plane et la couche de matériau réfléchissant une épaisseur sensiblement uniforme.

Plus particulièrement, le substrat peut être choisi parmi les verres, les céramiques, le graphite, les oxydes métalliques, les alumines, les silices, les silicates, notamment les aluminosilicates et les borosilicates, le talc, le mica, le mica synthétique, les métaux et leurs mélanges, cette liste n'étant pas limitative.

Le matériau réfléchissant peut comporter une couche de métal ou d'un composé métallique.

La couche de métal ou de composé métallique peut enrober ou non en totalité le substrat et la couche de métal peut être au moins partiellement recouverte par une couche d'un autre matériau, par exemple un matériau transparent. Il peut être préférable que la couche de métal ou de composé métallique enrobe en totalité, directement ou indirectement, c'est-à-dire avec interposition d'au moins une couche intermédiaire, métallique ou non, le substrat.

Le métal peut être choisi par exemple parmi Ag, Au, Cu, Al, Ni, Sn, Mg, Cr, Mo, Ti, Pt, Va, Rb, W, Zn, Ge, Te, Se et leurs alliages. Ag, Au, Al, Zn, Ni, Mo, Cr, Cu et leurs alliages (par exemple les bronzes et les laitons) sont des métaux préférés.

Dans le cas notamment de particules à substrat enrobé d'argent ou d'or, la couche métallique peut être présente à une teneur représentant par exemple de 0,1 à 50 % du poids total des particules, voire entre 1 et 20 %.

Des particules de verre recouvertes d'une couche métallique sont décrites notamment dans les documents JP-A-09188830, JP-A-10158450, JP-A-10158541, JP-A-07258460 et JP-A-05017710, dont le contenu est incorporé par référence.

Toujours à titre d'exemple de particules réfléchissantes comportant un substrat minéral enrobé d'une couche de métal, on peut citer également les particules comportant un substrat de borosilicate enrobé d'argent, encore appelées « nacres blanches ».

Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société. Des particules à substrat de verre sphérique revêtu ou non par un métal sont vendues sous la dénomination PRIZMALITE MICROSPHERE par la société PRIZMALITE INDUSTRIES.

Des particules à substrat métallique tel que l'aluminium, le cuivre, le bronze, en forme de plaquettes, sont vendues sous la dénomination commerciale STARBRITE par la société SILBERLINE et sous la dénomination VISIONAIRE par la société ECKART.

Les particules réfléchissantes quelle que soit leur forme, peuvent également être choisies parmi les particules à substrat synthétique enrobé au moins partiellement d'au moins une couche d'au moins un composé métallique, notamment un oxyde métallique, choisi par exemple parmi les oxydes de titane, notamment TiO₂, de fer notamment Fe₂O₃, d'étain, de chrome, le sulfate de baryum et les composés suivants : MgF₂, CrF₃, ZnS, ZnSe, SiO₂, Al₂O₃, MgO, Y₂O₃, SeO₃, SiO, HfO₂, ZrO₂, CeO₂, Nb₂O₅, Ta₂O₅, MoS₂ et leurs mélanges ou alliages.

A titre d'exemple de telles particules, on peut citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS® par la société ENGELHARD.

Conviennent également à l'invention, les pigments de la gamme METASHINE 1080R commercialisée par la société NIPPON SHEET GLASS CO. LTD. Ces pigments, plus particulièrement décrits dans la demande de brevet JP 2001-11340, sont des paillettes de verre C-GLASS comprenant 65 à 72 % de SiO₂, recouvertes d'une couche d'oxyde de titane de type rutile (TiO₂). Ces paillettes de verre ont une épaisseur moyenne de 1 micron et une taille moyenne de 80 microns soit un rapport en taille moyenne/épaisseur moyenne de 80. Elles présentent des reflets bleus, verts, jaunes ou de teinte argent selon l'épaisseur de la couche de TiO₂.

On peut encore citer les particules de dimension comprise entre 80 et 100 µm, comportant un substrat de mica synthétique (fluorophlogopite) revêtu de dioxyde de titane représentant 12% du poids total de la particule, vendues sous la dénomination PROMINENCE par la société NIHON KOKEN.

Les particules réfléchissantes peuvent encore être choisies parmi les particules formées d'un empilement d'au moins deux couches à indices de réfraction différents.

Ces couches peuvent être de nature polymérique ou métallique et notamment inclure au moins une couche polymérique.

Ainsi, les particules réfléchissantes peuvent être des particules dérivant d'un film polymérique multicouche.

De telles particules sont notamment décrites dans WO 99/36477, US 6 299 979 et US 6 387 498.

A titre illustratif des matériaux pouvant constituer les différentes couches de la structure multicouche, on peut citer, cette liste n'étant pas limitative: le naphthalate de polyéthylène (PEN) et ses isomères par exemple 2,6-, 1,4-, 1,5-, 2,7- et 2,3-PEN, les téréphthalates de polyalkylene, des polyimides, des polyéthérimides, des polystyrènes atactiques, des polycarbonates, des polyméthacrylates et des polyacrylates d'alkyle, du polystyrène syndiotactique (sPS), des poly-alpha-méthylstyrène syndiotactiques, du polydichlorostyrène syndiotactique, copolymères et mélange de ses polystyrènes, des dérivés de cellulose, des polymères polyalkylènes, des polymères fluorés, des polymères chlorés, des polysulfones, des polyéthersulfones, des polyacrylonitriles, des polyamides, des résines siliconées, des résines époxy, de l'acétate de polyvinyle, des polyéthers-amides, des résines ionomériques, des élastomères et polyuréthanes. Conviennent également des copolymères, par exemple copolymères de PEN (par exemple, copolymères de 2,6-, 1,4-, 1,5-, 2,7-, et/ou 2,3-acide naphtalène dicarboxylique ou ses esters avec (a) acide téréphtalique ou ses esters ; (b) l'acide isophtalique ou ses esters ; (c) acide phtalique ou ses esters ; (d) des alcanes glycols ; (e) des cycloalkanes glycols (par exemple le cyclohexane diméthanol diol) ; (f) des acides alcanes dicarboxyliques ; et/ou (g) des acides cycloalkanes dicarboxylique, des copolymères de téréphthalates de polyalkylène et des copolymères de styrène. En outre, chaque couche individuelle peut inclure des mélanges de deux ou plusieurs polymères ou copolymères précédents.

Le choix des matériaux destinés à constituer les différentes couches de la structure multicouche est bien entendu effectué de manière à conférer l'aspect réfléchissant souhaité aux particules ainsi formées.

Des particules réfléchissantes comportant un empilement d'au moins deux couches de polymères sont commercialisées par la société 3M sous la dénomination MIRROR GLITTER. Ces particules comportent des couches de 2,6-PEN et de polyméthacrylate de méthyle dans un rapport massique de 80/20. De telles particules sont décrites dans le brevet US 5 825 643.

### Nacres

Le deuxième agent de coloration peut comporter des nacres.

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres TIMICA, FLAMENCO et DUOCHROME (sur base de mica) commercialisées par la société ENGELHARD, les nacres TIMIRON commercialisées par la société MERCK, les nacres sur base de mica PRESTIGE commercialisées par la société ECKART et les nacres sur base de mica synthétique SUNSHINE commercialisées par la société SUN CHEMICAL.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fme (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

### Agents de coloration goniochromatiques

Le deuxième agent de coloration peut comporter au moins un agent de coloration goniochromatique pour créer, lorsque la composition est appliquée sur son support, un fond coloré dont la couleur change avec l'angle d'observation. Un agent de coloration goniochromatique au sens de la présente invention permet d'observer un changement de couleur, encore appelé « color flop », en fonction de l'angle d'observation, supérieur à celui que l'on peut rencontrer avec des nacres. On peut utiliser un seul agent de coloration goniochromatique pour une facilité de mise en oeuvre.

L'agent de coloration goniochromatique peut être choisi de manière à présenter un changement de couleur relativement important avec l'angle d'observation.

L'agent de coloration goniochromatique peut ainsi être choisi de telle sorte que l'on puisse observer, pour une variation de l'angle d'observation comprise entre 0° et 80° sous un éclairage à 45°, une variation de couleur ΔE de la composition cosmétique, mesurée dans l'espace colorimétrique CIE 1976, d'au moins 2.

L'agent de coloration goniochromatique peut également être choisi de telle sorte que l'on puisse observer, pour un éclairage à 45° et une variation de l'angle d'observation comprise entre 0° et 80°, une variation Dh de l'angle de teinte de la composition cosmétique, dans le plan CIE 1976, d'au moins 30° voire au moins 40° ou au moins 60°, voire encore d'au moins 100°.

L'agent de coloration goniochromatique peut être choisi par exemple parmi les structures multicouche interférentielles et les agents de coloration à cristaux liquides.

Dans le cas d'une structure multicouche, celle-ci peut comporter par exemple au moins deux couches, chaque couche, indépendamment ou non de la (ou les) autre(s) couche(s), étant réalisée par exemple à partir d'au moins un matériau choisi dans le groupe constitué par les matériaux suivants : MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolithe, alliages, polymères et leurs associations.

La structure multicouche peut présenter ou non, par rapport à une couche centrale, une symétrie au niveau de la nature chimique des couches empilées.

Des exemples de structures multicouche interférentielles symétriques utilisables dans des compositions réalisées conformément à l'invention sont par exemple les structures suivantes : Al/SiO₂/Al/SiO₂/Al, des pigments ayant cette structure étant commercialisés par la société DUPONT DE NEMOURS ; Cr/MgF₂/Al/MgF₂/Cr, des pigments ayant cette structure étant commercialisés sous la dénomination CHROMAFLAIR par la société FLEX ; MoS₂/SiO₂/Al/SiO₂/MoS₂ ; Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination SICOPEARL par la société BASF ; MoS₂/SiO₂/mica-oxyde/SiO₂/MoS₂ ; Fe₂O₃/SiO₂/mica-oxyde/SiO₂/Fe₂O₃ ; TiO₂/SiO₂/TiO₂ et TiO₂/Al₂O₃/TiO₂, SnO/TiO₂/SiO₂/TiO₂/SnO; Fe₂O₃/SiO₂/Fe₂O₃; SnO/mica/TiO₂/SiO₂/TiO₂/mica/SnO, des pigments ayant ces structures étant commercialisés sous la dénomination XIRONA par la société MERCK (Darmstadt). A titre d'exemple, ces pigments peuvent être les pigments de structure silice/oxyde de titane/oxyde d'étain commercialisés sous le nom XIRONA MAGIC par la société MERCK, les pigments de structure silice/oxyde de fer brun commercialisés sous le nom XIRONA INDIAN SUMMER par la société MERCK et les pigments de structure silice/oxyde de titane/mica/oxyde d'étain commercialisés sous le nom XIRONA CARRIBEAN BLUE par la société MERCK. On peut encore citer les pigments INFINITE COLORS de la société SHISEIDO. Selon l'épaisseur et la nature des différentes couches, on obtient différents effets. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

On peut encore utiliser des agents de coloration goniochromatiques à structure multicouche comprenant une alternance de couches polymériques par exemple du type naphtalate de polyéthylène et téréphtalate de polyéthylène. De tels agents sont notamment décrits dans WO-A-96/19347 et WO-A-99/36478.

On peut citer, à titre d'exemple de pigments à structure multicouche polymérique, ceux commercialisés par la société 3M sous la dénomination COLOR GLITTER.

Les agents de coloration à cristaux liquides comprennent par exemple des silicones ou des éthers de cellulose sur lesquels sont greffés des groupes mésomorphes.

Comme particules goniochromatiques à cristaux liquides, on peut utiliser par exemple celles vendues par la société CHENIX ainsi que celle commercialisées sous la dénomination HELICONE® HC par la société WACKER.

La composition peut en outre comporter des fibres goniochromatiques dispersées. De telles fibres peuvent par exemple présenter une taille comprise entre 50 µm et 700 µm, par exemple d'environ 300 µm.

En particulier, on peut utiliser des fibres interférentielles à structure multicouche. Des fibres à structure multicouche de polymères sont notamment décrites dans les documents EP-A-921217, EP-A-686858 et US-A-5472798. La structure multicouche peut comporter au moins deux couches, chaque couche, indépendamment ou non de la (ou les) autre(s) couche(s), étant réalisée en au moins un polymère de synthèse. Les polymères présents dans les fibres peuvent avoir un indice de réfraction allant de 1,30 à 1,82 et mieux allant de 1,35 à 1,75. Les polymères préférés pour constituer les fibres sont les polyesters tels que le polyéthylène téréphtalate, le polyéthylène naphtalate, le polycarbonate ; les polymères acryliques comme le polyméthacrylate de méthyle ; les polyamides.

Des fibres goniochromatiques à structure bicouche polyéthylène téréphtalate/nylon-6 sont commercialisées par la société TEIJIN sous la dénomination MORPHOTEX.

### AUTRES COMPOSANTS

### Solvants

La composition peut comporter au moins un solvant aqueux ou organique.

Lorsque la composition comprend un ou plusieurs solvants organiques, ces solvants peuvent être présents en une teneur allant de 0,1 % à 99 %, par rapport au poids total de la composition.

D'une manière générale, la quantité de solvant(s), notamment organique(s), dépendra de la nature du support sur lequel la composition est destinée à être appliquée.

Dans le cas d'un vernis à ongles, par exemple, le solvant organique pourra être présent dans la composition à une teneur allant par exemple de 30 à 99 % en poids et de préférence de 60 % à 90 % en poids, par rapport au poids total de la composition.

La composition peut comporter au moins un solvant organique choisi dans la liste suivante :
- les cétones liquides à température ambiante, tels que le méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les alcanes liquides à température ambiante tels que le décane, l'heptane, le dodécane, le cyclohexane ;

La composition peut aussi comprendre de l'eau ou un mélange d'eau et de solvants organiques hydrophiles couramment utilisés en cosmétique comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols. La composition peut, en outre, contenir des éthers en C₂ et des aldéhydes en C₂-C₄ hydrophiles. L'eau ou le mélange d'eau et de solvants organiques hydrophiles peut être présent dans la composition en une teneur allant par exemple de 0 % à 90 %, notamment 0,1 % à 90 % en poids et de préférence de 0 % à 60 % en poids, notamment 0,1 % à 60 % en poids, par rapport au poids total de la composition.

### Phase grasse

La composition, notamment lorsqu'elle est destinée à être appliquée sur les lèvres, peut comporter une phase grasse et notamment au moins un corps gras liquide à température ambiante (25 °C) et/ou un corps gras solide à température ambiante tel que les cires, les corps gras pâteux, les gommes, et leurs mélanges. La phase grasse peut, en outre, contenir des solvants organiques lipophiles.

La composition peut présenter par exemple une phase grasse continue, pouvant contenir moins de 5% d'eau, notamment moins de 1% d'eau par rapport à son poids total et en particulier être sous forme anhydre.

Comme corps gras liquides à température ambiante, appelés souvent « huiles », on peut citer : les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité, de lanoline, de lanoline acétylée ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; l'isonanoate d'isononyle, le lanolate d'isopropyle, le trimellilate de tridécyle, le malate de diisostéaryle ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges. Les huiles peuvent être présentes en une teneur allant de 0,01 à 90 %, et mieux de 0,1 à 85 % en poids, par rapport au poids total de la composition.

Les corps gras pâteux sont généralement des composés hydrocarbonés avec un point de fusion compris entre 25 et 60 °C, de préférence entre 30 et 45 °C, et/ou une dureté comprise entre 0,001 et 0,5 MPa, de préférence entre 0,005 et 0,4 MPa, comme les lanolines et leurs dérivés.

Les cires peuvent être solides à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En particulier, les cires peuvent présenter une température de fusion supérieure à 25 °C et mieux supérieure à 45 °C. Les cires peuvent être hydrocarbonées, fluorées et/ou siliconées et être d'origine végétale, minérale, animale et/ou synthétique. Comme cires utilisables on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone. La composition peut contenir de 0 à 50 % en poids de cires, par rapport au poids total de la composition, voire de 1 à 30 % en poids.

Les gommes pouvant être utilisées sont généralement des polydiméthylsiloxanes (PDMS) à haut poids moléculaire ou des gommes de cellulose ou des polysaccharides.

### Polymère filmogène

La composition peut encore comporter, par exemple, un polymère filmogène, notamment dans le cas d'un mascara ou d'un vernis à ongles. "Polymère filmogène" désigne un polymère apte à former à lui seul ou en présence d'un agent auxiliaire de filmification, un film continu et adhérent sur un support, notamment sur les matières kératiniques.

Parmi les polymères filmogènes utilisables dans une composition selon l'invention, on peut citer entre autres les polymères synthétiques, de type radicalaire ou de type polycondensat, les polymères d'origine naturelle, tels que la nitrocellulose ou les esters de cellulose, et leurs mélanges.

Les polymères filmogènes de type radicalaire peuvent être notamment des polymères ou des copolymères vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides comme les acides carboxyliques insaturés α,β-éthyléniques tels que l'acide acrylique, l'acide méthacrylique, l'acide crotonique, l'acide maléique, l'acide itaconique.

Les polymères filmogènes vinyliques peuvent également résulter de l'homopolymérisation ou de la copolymérisation de monomères choisis parmi les esters vinyliques comme l'acétate de vinyle, le néodécanoate de vinyle, le pivalate de vinyle, le benzoate de vinyle et le t-butyl benzoate de vinyle et les monomères styrèniques comme le styrène et l'alpha-méthyl styrène.

Parmi les polycondensats filmogènes, on peut citer les polyuréthanes, les polyesters, les polyesters amides, les polyamides, et les polyurées, cette liste n'étant pas limitative.

Les polymères d'origine naturelle, éventuellement modifiés, peuvent être choisis parmi la résine shellac, la gomme de sandaraque, les dammars, les élémis, les copals, les polymères cellulosiques, tels que la nitrocellulose, l'éthylcellulose ou les esters de nitrocellulose choisis, par exemple, parmi l'acétate de cellulose, l'acétobutyrate de cellulose, l'acétopropionate de cellulose, et leurs mélanges.

Le polymère filmogène peut être présent sous la forme de particules solides en dispersion aqueuse ou huileuse, connue généralement sous le nom de latex ou pseudolatex. Le polymère filmogène peut comporter une ou plusieurs dispersions stables de particules de polymères généralement sphériques d'un ou plusieurs polymères, dans une phase grasse liquide physiologiquement acceptable. Ces dispersions sont généralement appelées NAD (Non-Aqueous Dispersion) de polymère par opposition à des latex qui sont des dispersions aqueuses de polymère. Ces dispersions peuvent notamment se présenter sous forme de nanoparticules de polymères en dispersion stable dans ladite phase grasse. Les nanoparticules sont de préférence d'une taille comprise entre 5 et 600 nm. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la société AVECIA-NEORESINS, DOW LATEX 432® par la société DOW CHEMICAL, DAITOSOL 5000 AD® par la société DAITO KASEI KOGYO; ou encore les dispersions aqueuses de polyuréthane vendues sous les dénominations NEOREZ R-981®, NEOREZ R-974® par la société AVECIA-NEORESINS, les AVALURE UR-405®, AVALURE UR-410®, AVALURE UR-425®, AVALURE UR-450®, SANCURE 875®, SANCURE 861®, SANCURE 878®, SANCURE 2060® par la société GOODRICH, IMPRANIL 85® par la société BAYER, AQUAMERE H-1511® par la société HYDROMER ; les sulfopolyesters vendus sous le nom de marque Eastman AQ par la société Eastman Chemical Products.

La composition selon l'invention peut comprendre également un agent auxiliaire de filmification favorisant la formation d'un film avec le polymère filmogène.

### Charges

La composition peut comprendre en outre des charges. Par « charges », on désigne des particules de toute forme, insolubles dans le milieu de la composition, quelle que soit la température à laquelle la composition est fabriquée. Ces charges peuvent servir notamment à modifier la rhéologie ou la texture de la composition. La nature et la quantité des corps solides sont fonction des propriétés mécaniques et des textures recherchées.

A titre d'exemple de charges, on peut citer, entre autres, le talc, le mica, la silice, le kaolin, la séricite, les poudres de polyamide, de polyéthylène, de polytétrafluoroéthylène, de polyméthacrylate de méthyle, de polyuréthane, les poudres d'amidon et les billes de résine de silicone.

### Matière colorante additionnelle

La composition peut comprendre une matière colorante additionnelle, différente du pigment composite utilisé dans la présente invention.

La matière colorante additionnelle peut être choisie parmi les pigments minéraux, les pigments organiques, les colorants liposolubles ou hydrosolubles.

Les pigments minéraux peuvent être blancs ou colorés, enrobés ou on. On peut citer le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Les pigments peuvent représenter de 0 à 40 %, de préférence de 1 à 35 %, et mieux de 2 à 25 % du poids total de la composition.

Parmi les pigments organiques, on peut citer le noir de carbone, les pigments de type D & C, et les laques à base de carmin de cochenille, de baryum, strontium, calcium, aluminium.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Les colorants hydrosolubles sont par exemple le jus de betterave et le bleu de méthylène.

Les colorants peuvent représenter de 0,1 à 20 % du poids de la composition et mieux de 0,1 à 6 % (si présents).

### Autres ingrédients

La composition peut comporter au moins un actif cosmétique ou dermatologique. Comme actifs cosmétiques, dermatologiques, hygiéniques ou pharmaceutiques, utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, anti-rides...). Ces actifs peuvent être utilisés par exemple à des concentrations de 0 à 20 % et notamment de 0,001 à 15 % par rapport au poids total de la composition.

La composition cosmétique peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants les filtres UV, les colorants ou leurs mélanges.

La composition selon l'invention peut comprendre, selon le type d'application envisagée, les constituants classiquement utilisés dans les domaines considérés, qui sont présents en une quantité appropriée à la forme galénique souhaitée.

### Formes galéniques

La composition peut se présenter sous diverses formes, en fonction de sa destination. La composition peut ainsi se présenter sous toute forme galénique normalement utilisée pour une application topique et notamment sous forme anhydre, sous forme d'une solution huileuse ou aqueuse, d'un gel huileux ou aqueux, d'une émulsion huile dans eau, eau dans huile, cire dans eau ou eau dans cire, d'une émulsion multiple, d'une dispersion d'huile dans de l'eau grâce à des vésicules situés à l'interface huile/eau.

La composition peut se présenter sous forme de produit coulé, notamment de stick dans le cas d'un rouge à lèvres ou d'un produit de soin des lèvres.

La composition peut encore se présenter sous diverses autres formes, par exemple d'un liquide plus ou moins visqueux, d'un gel ou d'une pâte.

La composition peut encore se présenter sous la forme d'un solide, par exemple un pain à humidifier au moment de l'utilisation, de manière à lui permettre de se déliter.

La composition cosmétique peut constituer une composition de maquillage, entre autres, un rouge à lèvres, un gloss liquide, une pâte de rouge à lèvres, un fard à joues, un crayon à lèvres, un fond de teint solide ou fluide, un produit anti-cernes ou de contour des yeux, un eye-liner, un mascara, un vernis à ongles, une ombre à paupières, un produit de maquillage du corps ou des cheveux ou encore un produit solaire ou de coloration de la peau.

L'invention a ainsi encore pour objet un rouge à lèvres, liquide ou solide, comportant une composition telle que définie plus haut.

L'invention a encore pour objet un fond de teint comportant une composition telle que définie plus haut.

L'invention a encore pour objet un vernis à ongles comportant une composition telle que définie plus haut.

L'invention a encore pour objet un mascara comportant une composition telle que définie plus haut.

L'invention a encore pour objet un produit de coloration des fibres capillaires comportant une composition telle que définie plus haut.

L'invention a encore pour objet un procédé de maquillage de la peau, des lèvres ou des phanères, dans lequel on applique sur la peau, les lèvres ou les phanères une composition telle que définie plus haut.

### EXEMPLES

On a réalisé, à titre illustratif, des compositions cosmétiques comportant des premier et deuxième agents de coloration avec les formulations suivantes, ces compositions étant préparées selon les procédés de préparation classiquement utilisés en cosmétique.

### Exemples 1, 2 et exemple comparatif 1 : Rouges à lèvres

### Exemple 1 :

Un rouge à lèvres de composition suivante conforme à l'invention a été préparé (quantités exprimées en % en poids par rapport au poids total de la composition) :

| | |
|---|---|
| Cire de polyéthylène | 10 |
| Néopentanoate d'octyldodécyle | 47,1 |
| Polybutène hydrogéné | 20 |
| Phényl triméthylsiloxy trisiloxane | 15 |
| Pigment goniochromatique (SICOPEARL Fantastico Green (BASF)) | 5,5 |
| Pigment composite silice/D&C Red N° 7¹ | 2,4 |

| | |
|---|---|
| ¹Pigment composite constitué de 50 parts en poids de D&C Red N° 7 pour 100 parts d'un noyau organique de silice de taille moyenne 15 nm et de surface spécifique 200 m²/g et réalisé avec un liant polyméthylhydrogénosiloxane. | |

### Exemple 2 :

Un rouge à lèvres de composition suivante conforme à l'invention a été préparé (quantités exprimées en % en poids par rapport au poids total de la composition) :

| | |
|---|---|
| Cire de polyéthylène | 10 |
| Néopentanoate d'octyldodécyle | 47,1 |
| Polybutène hydrogéné | 20 |
| Phényl triméthylsiloxy trisiloxane | 15 |
| Pigment goniochromatique (SICOPEARL Fantastico Green (BASF)) | 5,5 |
| Pigment composite TᵢO₂/D&C Red N° 7² | 2,4 |

| | |
|---|---|
| ²Pigment composite constitué de 50 parts en poids de D&C Red N° 7 pour 100 parts d'un noyau inorganique de dioxyde de titane de taille moyenne 20 nm et de surface spécifique 50 m²/g et réalisé avec un liant polyméthylhydrogénosiloxane. | |

### Exemple comparatif 1 :

Un rouge à lèvres de composition suivante non conforme à l'invention car dépourvu de pigment composite, a été préparé (quantités exprimées en % en poids par rapport au poids total de la composition) :

| | |
|---|---|
| Cire de polyéthylène | 10 |
| Néopentanoate d'octyldodécyle | 48,7 |
| Polybutène hydrogéné | 20 |
| Phényl triméthylsiloxy trisiloxane | 15 |
| Pigment goniochromatique (SICOPEARL Fantastico Green (BASF)) | 5,5 |
| Pigment organique D&C Red N° 7 | 0,8 |

On a déposé les rouges à lèvres des exemples 1, 2 et de l'exemple comparatif 1 sur un fond noir d'une carte de contraste de type 24/5 d'ERICSEN comportant un fond noir et un fond blanc, avec une épaisseur de 100 µm au moyen d'un étaleur automatique.

Le trajet de couleur des différents échantillons, dans le plan a*b* de l'espace colorimétrique CIE 1976, a ensuite été mesuré en réflexion à l'aide du spectrogoniocolorimètre GC-5000 de NIPPON DENSHOKU KOGYO, avec un angle d'incidence à 45° et un angle d'observation variant de 0 à 70°.

| Angle d'observation (°) | Exemple 1 | | Exemple 2 | | Exemple comparatif 1 | |
|---|---|---|---|---|---|---|
| | a* | b* | a* | b* | a* | b* |
| 0 | 12,8 | 5,2 | 16,5 | 3,9 | 14,3 | 7,2 |
| 10 | 13,5 | 2,8 | 17,4 | 2,0 | 13,8 | 4,1 |
| 20 | 14,9 | -0,8 | 18,7 | -0,8 | 14,8 | 0,3 |
| 30 | 16,7 | -3,6 | 20,3 | -3,6 | 16,0 | -3,1 |
| 40 | 5,1 | -2,0 | 9,2 | -5,9 | 4,8 | -3,3 |
| 50 | -16,6 | -13,0 | -19,7 | -14,6 | 1,5 | -4,8 |
| 60 | 17,3 | -2,3 | 21,3 | -2,5 | 18,3 | -2,9 |
| 70 | 19,6 | 1,1 | 20,0 | 1,1 | 19,2 | 0,5 |

L'exemple comparatif 1 ne fait pas ressortir la composante verte du pigment et a un effet « color flop » réduit par rapport à celui des exemples 1 et 2.

### Exemple 3 : Fond de teint liquide

On a préparé le fond de teint liquide conforme à l'invention ayant la composition suivante (quantités exprimées en % en poids par rapport au poids total de la composition).

| | |
|---|---|
| Diméthicone copolyol | 5 |
| Diméthicone | 4 |
| Cyclométhicone | 15 |
| Isododécane | 10 |
| Gel de bentone | 10 |
| Poudre de nylon | 2 |
| Poudre de PMMA | 2 |
| Oxyde de fer jaune | 4 |
| Pigment composite TiO₂/D&C Red N° 7 | 1 |
| Pigment mica/oxychlorure de bismuth/oxyde de fer brun⁴ | 5 |
| Eau | 31,2 |
| Butylène glycol | 10 |
| Sulfate de magnésium | 0,8 |

| | |
|---|---|
| ⁴ Chroma-Lite Brown commercialisé par ENGELHARD. | |

### Exemple 4 : Vernis à ongles

On a préparé le vernis à ongles conforme à l'invention ayant la composition suivante (quantités exprimées en % en poids par rapport au poids total de la composition).

| | |
|---|---|
| Nitrocellulose | 19 |
| Sulfonamide de N-éthyl-o,p-toluène | 6 |
| Tributyle d'acétyle citrate | 6 |
| Agent rhéologique (hectorite) | 1,2 |
| Pigment composite silice/D&C Red N°7 | 3 |
| Particules de verre enrobées d'argent⁵ | 6 |
| Isopropanol | 8 |
| Acétate d'éthyle/Acétate de butyle | qsp 100 |

| | |
|---|---|
| ⁵ Métashine REFSX 2040 PS commercialisé par la société TOYAL. | |

On peut observer un effet de brillance ponctuelle lié à la présence des particules de verre enrobées d'argent.

Bien entendu, l'invention n'est pas limitée aux exemples qui viennent d'être donnés.

Dans toute la description, y compris les revendications, l'expression « comportant un » doit être comprise comme étant synonyme de « comportant au moins un », sauf si le contraire est spécifié.

Les intervalles donnés doivent être compris bornes incluses, sauf si le contraire est spécifié.

## Revendications

1. Composition destinée à être appliquée sur la peau, les lèvres et/ou les phanères, comportant :
- entre 0,1 % et 20 % en poids, par rapport au poids total de la composition, d'au moins un premier agent de coloration dans une quantité suffisante pour colorer la composition, comportant des particules d'au moins un pigment composite non interférentiel, ces particules comportant :
- un noyau inorganique,
- un enrobage au moins partiel d'au moins une matière colorante organique,
cette composition comportant en outre :
- entre 0,1 % et 50 % en poids, par rapport au poids total de la composition, d'au moins un deuxième agent de coloration dans une quantité suffisante pour produire dans la composition un effet optique spécifique perceptible à l'oeil nu,
le deuxième agent de coloration comportant des particules réfléchissantes, des nacres et/ou un agent de coloration goniochromatique.

2. Composition selon la revendication 1, **caractérisée par le fait que** l'effet optique spécifique est un effet de brillance ponctuelle.

3. Composition selon la revendication 1, **caractérisée par le fait que** l'effet optique spécifique est un effet goniochromatique.

4. Composition selon la revendication 1, **caractérisée par le fait que** l'effet optique spécifique est un effet tacheté.

5. Composition selon l'une quelconque des revendications précédentes, **caractérisée par le fait que** la teneur en premier agent de coloration dans la composition est comprise entre 0,1 % et 10 % en poids, par rapport au poids total de la composition.

6. Composition selon la revendication précédente, **caractérisée par le fait que** la teneur en premier agent de coloration dans la composition est comprise entre 0,5 % et 5 % en poids, par rapport au poids total de la composition.

7. Composition selon l'une quelconque des revendications précédentes, **caractérisée par le fait que** la teneur en deuxième agent de coloration dans la composition est comprise entre 0,5 % et 40 % en poids, par rapport au poids total de la composition.

8. Composition selon la revendication précédente, **caractérisée par le fait que** la teneur en deuxième agent de coloration dans la composition est comprise entre 1 % et 20 % en poids, par rapport au poids total de la composition.

9. Composition selon l'une quelconque des revendications précédentes, **caractérisée par le fait que** le rapport en poids du deuxième agent de coloration au premier agent de coloration est compris entre 0,1 et 50.

10. Composition selon la revendication précédente, **caractérisée par le fait que** le rapport en poids du deuxième agent de coloration au premier agent de coloration est compris entre 0,5 et 30.

11. Composition selon la revendication précédente, **caractérisée par le fait que** le rapport en poids du deuxième agent de coloration au premier agent de coloration est compris entre 1 et 10.

12. Composition selon l'une quelconque des revendications précédentes, **caractérisée par le fait que** la matière colorante organique comporte au moins un pigment organique.

13. Composition selon l'une quelconque des revendications précédentes, **caractérisée par le fait que** la matière colorante organique comporte au moins une laque organique.

14. Composition selon l'une quelconque des revendications précédentes, **caractérisée par le fait que** le noyau inorganique présente une taille moyenne comprise entre 1 nm et 100 nm.

15. Composition selon la revendication précédente, **caractérisée par le fait que** le noyau inorganique présente une taille moyenne comprise entre 5 nm et 75 nm.

16. Composition selon la revendication précédente, **caractérisée par le fait que** le noyau inorganique présente une taille moyenne comprise entre 10 nm et 50 nm.

17. Composition selon l'une quelconque des revendications précédentes, **caractérisée par le fait que** le noyau inorganique présente une surface spécifique comprise entre 1 m²/g et 1000 m²/g.

18. Composition selon la revendication précédente, **caractérisée par le fait que** la surface spécifique du noyau inorganique est comprise entre 10 m²/g et 600 m²/g

19. Composition selon la revendication précédente, **caractérisée par le fait que** la surface spécifique du noyau inorganique est comprise entre 20 m²/g et 400 m²/g.

20. Composition selon l'une quelconque des revendications précédentes, **caractérisée par le fait que** le noyau inorganique comporte un matériau choisi parmi un sel métallique, un oxyde métallique, une alumine, un verre, une céramique, un graphite, une silice, un silicate, un mica synthétique, et un de leurs mélanges.

21. Composition selon la revendication précédente, **caractérisée par le fait que** le noyau inorganique comporte un oxyde métallique choisi parmi un oxyde de titane, de fer, de cérium, de zirconium, de zinc, d'aluminium, de bleu ferrique, et de chrome.

22. Composition selon la revendication précédente, **caractérisée par le fait que** le noyau inorganique comporte un oxyde métallique choisi parmi un oxyde de titane, de fer, de cérium, de zirconium, de zinc et d'aluininium.

23. Composition selon la revendication précédente, **caractérisée par le fait que** le noyau inorganique comporte du dioxyde de titane.

24. Composition selon la revendication 20, **caractérisée par le fait que** le noyau inorganique comporte au moins un silicate choisi parmi un aluminosilicate et un borosilicate.

25. Composition selon la revendication 20, **caractérisée par le fait que** le noyau inorganique comporte de la silice.

26. Composition selon l'une quelconque des revendications précédentes, **caractérisée par le fait que** la proportion massique de matière colorante organique est comprise entre 10 et 500 parts en poids pour 100 parts en poids du noyau inorganique.

27. Composition selon la revendication précédente, **caractérisée par le fait que** la proportion massique de la matière colorante organique est comprise entre 20 et 250 parts en poids pour 100 parts en poids du noyau inorganique.

28. Composition selon la revendication précédente, **caractérisée par le fait que** la proportion massique de la matière colorante organique est comprise entre 40 et 125 parts en poids pour 100 parts en poids du noyau inorganique.

29. Composition selon l'une quelconque des revendications précédentes, **caractérisée par le fait que** la matière colorante organique est choisie parmi un carmin de cochenille, un pigment organique de colorant azoïque, anthraquinonique, indigoïde, xanthénique, pyrénique, quinolinique, de triphénylméthane, de fluorane, une laque organique, un sel insoluble de sodium, de potassium, de calcium, de baryum, d'aluminium, de zirconium, de strontium, de titane, de colorant acide.

30. Composition selon la revendication précédente, **caractérisée par le fait que** le colorant acide est choisi parmi un colorant azoïque, anthraquinonique, indigoïde, xanthénique, pyrénique, quinolinique, de triphénylméthane, de fluorane, et tout autre colorant comportant au moins un groupe acide carboxylique ou sulfonique.

31. Composition selon l'une quelconque des revendications précédentes, **caractérisée par le fait que** la matière colorante organique comporte une laque organique supportée par un support organique comportant au moins une colophane ou du benzoate d'aluminium.

32. Composition selon l'une quelconque des revendications précédentes, **caractérisée par le fait que** la matière colorante organique comporte un pigment organique ayant l'une des dénominations suivantes : D&C Blue n° 4, D&C Brown n° 1, D&C Green n° 5, D&C Green n° 6, D&C Orange n°. 4, D&C Orange n° 5, D&C Orange n° 10, D&C Orange n° 11, D&C Red n° 6, D&C Red n° 7, D&C Red n° 17, D&C Red n° 21, D&C Red n° 22, D&C Red n° 27, D&C Red n° 28, D&C Red n° 30, D&C Red n° 31, D&C Red n° 33, D&C Red n° 34, D&C Red n° 36, D&C Violet n° 2, D&C Yellow n° 7, D&C Yellow n° 8, D&C Yellow n° 10, D&C Yellow n° 11, FD&C Blue n° 1, FD&C Green n° 3, FD&C Red n° 40, FD&C Yellow n° 5, FD&C Yellow n° 6.

33. Composition selon l'une quelconque des revendications précédentes, **caractérisée par le fait que** la matière colorante organique comporte une laque organique ayant l'une des dénominations suivantes : D&C Red n° 2 Aluminium lake, D&C Red n° 3 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 19 Aluminium lake, D&C Red n° 19 Barium lake, D&C Red n° 19 Zirconium lake, D&C Red n° 21 Aluminium lake, D&C Red n° 21 Zirconium lake, D&C Red n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n° 27 Zirconium lake, D&C Red n° 28 Aluminium lake, D&C Red n° 30 lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Blue n° 1 Aluminium lake, D&C Green n° 3 Aluminium lake, D&C Orange n° 4 Aluminium lake, D&C Orange n° 5 Aluminium lake, D&C Orange n° 5 Zirconium lake, D&C Orange n° 10 Aluminium lake, D&C Orange n° 17 Barium lake, D&C Yellow n° 5 Aluminium lake, D&C Yellow n° 5 Zirconium lake, D&C Yellow n° 6 Aluminium lake, D&C Yellow n° 7 Zirconium lake, D&C Yellow n° 10 Aluminium lake, FD&C Blue n° 1 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

34. Composition selon l'une quelconque des revendications précédentes, **caractérisée par le fait que** le pigment composite comporte au moins un liant contribuant à la fixation de la matière colorante organique sur le noyau inorganique.

35. Composition selon la revendication 34, **caractérisée par le fait que** le liant est inorganique.

36. Composition selon la revendication précédente, **caractérisée par le fait que** le liant comporte au moins l'un d'un composé siliconé, d'un composé polymérique, d'un composé oligomérique et similaire comportant au moins l'un d'un organosilane, un organosilane d'un organosilane d'un fluoroalkylé, d'un polysiloxane, d'un agent couplant, et d'un mélange de ceux-ci.

37. Composition selon la revendication précédente, **caractérisée par le fait que** l'agent couplant est à base de silane, de titanate, d'aluminate et/ou de zirconate, ou d'un mélange de ceux-ci.

38. Composition selon la revendication 36, **caractérisée par le fait que** le liant comporte au moins un composé siliconé.

39. Composition selon la revendication précédente, **caractérisée par le fait que** le liant comporte du polyméthylhydrogénosiloxane.

40. Composition selon l'une quelconque des revendications précédentes, **caractérisée par le fait qu'**elle est dépourvue de particules de dioxyde de titane non enrobées.

41. Composition selon l'une quelconque des revendications précédentes, **caractérisée par le fait que** le deuxième agent de coloration présente une structure multicouche.

42. Composition selon la revendication précédente, **caractérisée par le fait que** le deuxième agent de coloration comporte un substrat comportant au moins l'un d'un métal, d'un oxyde métallique et d'un polymère.

43. Composition selon l'une quelconque des revendications précédentes, **caractérisée par le fait que** le deuxième agent de coloration comporte des nacres.

44. Composition selon la revendication précédente, **caractérisée par le fait que** le deuxième agent de coloration comporte au moins l'un d'un mica, d'un mica synthétique, d'un talc, d'une silice et d'une alumine.

45. Composition selon l'une quelconque des revendications précédentes, **caractérisée par le fait que** le deuxième agent de coloration comporte des particules à base de verre.

46. Composition selon l'une quelconque des revendications précédentes, **caractérisée par le fait que** le deuxième agent de coloration comporte au moins un agent de coloration goniochromatique.

47. Composition selon l'une quelconque des revendications précédentes, **caractérisée par le fait que** le deuxième agent de coloration comporte des particules réfléchissantes.

48. Composition selon l'une quelconque des revendications précédentes, **caractérisée par le fait que** le deuxième agent de coloration comporte des particules métallisées en surface.

49. Composition selon la revendication 47, **caractérisée par le fait que** les particules réfléchissantes comprennent des particules comportant un substrat synthétique enrobé au moins partiellement par au moins une couche d'au moins un métal ou composé métallique.

50. Composition selon l'une quelconque des revendications précédentes, **caractérisée par le fait qu'**elle se présente sous une forme solide.

51. Composition selon l'une quelconque des revendications précédentes, **caractérisée par le fait qu'**elle se présente sous forme liquide, pâteuse ou gélifiée.

52. Composition selon l'une quelconque des revendications 1 à 51, **caractérisée en ce qu'**il s'agit d'un rouge à lèvres.

53. Composition selon l'une quelconque des revendications 1 à 51, **caractérisée en ce qu'**il s'agit d'un fond de teint.

54. Composition selon l'une quelconque des revendications 1 à 51, **caractérisée en ce qu'**il s'agit d'un vernis à ongles.

55. Composition selon l'une quelconque des revendications 1 à 51, **caractérisée en ce qu'**il s'agit d'un mascara.

56. Composition selon l'une quelconque des revendications 1 à 51, **caractérisée en ce qu'**il s'agit d'un produit de coloration des fibres capillaires.

## Patentansprüche

1. Zusammensetzung zur Auftragung auf die Haut, Lippen und/oder Hautanhangsgebilde, umfassend:
- zwischen 0,1 und 20 Gew.-%, im Verhältnis zum Gesamtgewicht der Zusammensetzung, wenigstens eines ersten Farbstoffes in ausreichender Menge, um die Zusammensetzung zu färben, umfassend Partikel wenigstens eines Verbundpigments ohne Interferenz, wobei diese Partikel:
- einen anorganischen Kern,
- eine wenigstens teilweise Beschichtung mit wenigstens einem organischen färbenden Material umfassen,
- wobei diese Zusammensetzung darüber hinaus:
- zwischen 0,1 und 50 Gew.-%, im Verhältnis zum Gesamtgewicht der Zusammensetzung, wenigstens eines zweiten Farbstoffes in ausreichender Menge umfasst, um in der Zusammensetzung einen spezifischen, optischen, mit bloßem Auge sichtbaren Effekt hervorzurufen,
wobei der zweite Farbstoff reflektierende Partikel, Perlmutt und/oder einen goniochromatischen Farbstoff umfasst.

2. Zusammensetzung nach Anspruch 1, **dadurch gekennzeichnet, dass** der spezifische optische Effekt ein punktueller Glanzeffekt ist.

3. Zusammensetzung nach Anspruch 1, **dadurch gekennzeichnet, dass** der spezifische optische Effekt ein goniochromatischer Effekt ist.

4. Zusammensetzung nach Anspruch 1, **dadurch gekennzeichnet, dass** der spezifische optische Effekt ein Fleckeneffekt ist.

5. Zusammensetzung nach einem der vorhergehenden Ansprüche, **dadurch gekennzeichnet, dass** der Gehalt an erstem Farbstoff in der Zusammensetzung zwischen 0,1 und 10 Gew.-%, im Verhältnis zum Gesamtgewicht der Zusammensetzung beträgt.

6. Zusammensetzung nach dem vorhergehenden Anspruch, **dadurch gekennzeichnet, dass** der Gehalt an erstem Farbstoff in der Zusammensetzung zwischen 0,5 und 5 Gew.-%, im Verhältnis zum Gesamtgewischt der Zusammensetzung beträgt.

7. Zusammensetzung nach einem der vorhergehenden Ansprüche, **dadurch gekennzeichnet, dass** der Gehalt an zweitem Farbstoff in der Zusammensetzung zwischen 0,5 und 40 Gew.-%, im Verhältnis zum Gesamtgewicht der Zusammensetzung beträgt.

8. Zusammensetzung nach dem vorhergehenden Anspruch, **dadurch gekennzeichnet, dass** der Gehalt an zweitem Farbstoff in der Zusammensetzung zwischen 1 und 20 Gew.-%, im Verhältnis zum Gesamtgewicht der Zusammensetzung beträgt.

9. Zusammensetzung nach einem der vorhergehenden Ansprüche, **dadurch gekennzeichnet, dass** das Gewichtsverhältnis des zweiten Farbstoffes zum ersten Farbstoff zwischen 0,1 und 50 liegt.

10. Zusammensetzung nach dem vorhergehenden Anspruch, **dadurch gekennzeichnet, dass** das Gewichtsverhältnis des zweiten Farbstoffes zum ersten Farbstoff zwischen 0,5 und 30 liegt.

11. Zusammensetzung nach dem vorhergehenden Anspruch, **dadurch gekennzeichnet, dass** das Gewichtsverhältnis des zweiten Farbstoffes zum ersten Farbstoff zwischen 1 und 10 liegt.

12. Zusammensetzung nach einem der vorhergehenden Ansprüche, **dadurch gekennzeichnet, dass** das organische färbende Material wenigstens ein organisches Pigment umfasst.

13. Zusammensetzung nach einem der vorhergehenden Ansprüche, **dadurch gekennzeichnet, dass** das organische färbende Material wenigstens einen organischen Lack umfasst.

14. Zusammensetzung nach einem der vorhergehenden Ansprüche, **dadurch gekennzeichnet, dass** der anorganische Kern eine durchschnittliche Größe zwischen 1 nm und 100 nm aufweist.

15. Zusammensetzung nach dem vorhergehenden Anspruch, **dadurch gekennzeichnet, dass** der anorganische Kern eine durchschnittliche Größe zwischen 5 nm und 75 nm aufweist.

16. Zusammensetzung nach dem vorhergehenden Anspruch, **dadurch gekennzeichnet, dass** der anorganische Kern eine durchschnittliche Größe zwischen 10 nm und 50 nm aufweist.

17. Zusammensetzung nach einem der vorhergehenden Ansprüche, **dadurch gekennzeichnet, dass** der anorganische Kern eine spezifische Oberfläche zwischen 1 m²/g und 1000 m²/g aufweist.

18. Zusammensetzung nach dem vorhergehenden Anspruch, **dadurch gekennzeichnet, dass** die spezifische Oberfläche des anorganischen Kerns zwischen 10 m²/g und 600 m²/g liegt.

19. Zusammensetzung nach dem vorhergehenden Anspruch, **dadurch gekennzeichnet, dass** die spezifische Oberfläche des anorganischen Kerns zwischen 20 m²/g und 400 m²/g liegt.

20. Zusammensetzung nach dem vorhergehenden Anspruch, **dadurch gekennzeichnet, dass** der anorganische Kern ein Material umfasst, das aus der Gruppe bestehend aus einem Metallsalz, einem Metalloxyd, einem Aluminiumoxyd, einem Glas, einer Keramik, einem Graphit, einem Siliziumdioxyd, einem Silikat, einem künstlichen Glimmer und einer ihrer Mischungen ausgewählt ist.

21. Zusammensetzung nach dem vorhergehenden Anspruch, **dadurch gekennzeichnet, dass** der anorganische Kern ein Metalloxyd umfasst, das in der Gruppe bestehend aus einem Titan- , Eisen-, Cer-, Zirconium-, Zink-, Aluminium-, Eisenblau-, und Chromoxyd ausgewählt ist.

22. Zusammensetzung nach dem vorhergehenden Anspruch, **dadurch gekennzeichnet, dass** der anorganische Kern ein Metalloxyd umfasst, das in der Gruppe bestehend aus einem Titan- , Eisen-, Cer-, Zirconium-, Zink- und Aluminiumoxyd ausgewählt ist.

23. Zusammensetzung nach dem vorhergehenden Anspruch, **dadurch gekennzeichnet, dass** der anorganische Kern Titandioxyd umfasst.

24. Zusammensetzung nach Anspruch 20, **dadurch gekennzeichnet, dass** der anorganische Kern wenigstens ein Silikat umfasst, das in einer Gruppe bestehend aus einem Aluminiumsilikat und einem Borosilikat ausgewählt ist.

25. Zusammensetzung nach Anspruch 20, **dadurch gekennzeichnet, dass** der anorganische Kern Siliziumdioxyd umfasst.

26. Zusammensetzung nach einem der vorhergehenden Ansprüche, **dadurch gekennzeichnet, dass** der Massenanteil an organischem, färbendem Material zwischen 10 und 500 Gew.-%, bei 100 Gew.-% des anorganischen Kerns liegt.

27. Zusammensetzung nach dem vorhergehenden Anspruch, **dadurch gekennzeichnet, dass** der Massenanteil an organischem, färbendem Materials zwischen 20 und 250 Gew.-%, bei 100 Gew.-% des anorganischen Kerns liegt.

28. Zusammensetzung nach dem vorhergehenden Anspruch, **dadurch gekennzeichnet, dass** der Massenanteil an organischem, färbendem Material zwischen 40 und 125 Gew.-%, bei 100 Gew.-% des anorganischen Kerns liegt.

29. Zusammensetzung nach einem der vorhergehenden Ansprüche, **dadurch gekennzeichnet, dass** das organische färbende Material in einer Gruppe bestehend aus einem Cochenille-karmin, einem organischen Pigment eines Azo-, Antrachinon-, Indigo-, Xanthen-, Pyren-, Chinolin-, Triphenylmethan, Fluoranfarbstoffes, einem organischen Lack, einem unlöslichen Natrium-, Kalium-, Calcium-, Barium-, Aluminium-, Zirconium-, Strontium-, Titan, Säurefarbstoffsalz ausgewählt ist.

30. Zusammensetzung nach dem vorhergehenden Anspruch, **dadurch gekennzeichnet, dass** der Säurefarbstoff in einer Gruppe bestehend aus einem Azo-, Antrachinon-, Indigo-, Xanthen-, Pyren-, Chinolin-, Triphenylmethan, Fluoranfarbstoff und jedem anderen Farbstoff, der wenigstens eine Carboxyl- oder Sulfonsäuregruppe umfasst, ausgewählt ist.

31. Zusammensetzung nach einem der vorhergehenden Ansprüche, **dadurch gekennzeichnet, dass** der organische färbende Farbstoff einen organischen Lack umfasst, der durch einen mindestens ein Kolophonium oder Aluminiumbenzoat umfassenden organischen Träger getragen ist.

32. Zusammensetzung nach einem der vorhergehenden Ansprüche, **dadurch gekennzeichnet, dass** das organische färbende Material ein organisches Pigment mit einer der folgenden Bezeichnungen umfasst: D&C Blue n°4, D&C Brown n°1, D&C Green n°5, D&C Green n°6, D&C Orange n°4, D&C Orange n°5, D&C Orange n°10, D&C Orange n° 11, D&C Red n°6, D&C Red n°7, D&C Red n°17, D&C Red n°21, D&C Red n°22, D&C Red n° 27, D&C Red n°28, D&C Red n°30, D&C Red n° 31, D&C Red n° 33, D&C Red n° 34, D&C Red n° 36, D&C Violet n° 2, D&C Yellow n° 7, D&C Yellow, n° 8, D&C Yellow n° 10, D&C Yellow n° 11, FD&C Blue n°1, FD&C Green n°3, FD&C Red n° 40, FD&C Yellow n° 5, FD&C Yellow n° 6.

33. Zusammensetzung nach einem der vorhergehenden Ansprüche, **dadurch gekennzeichnet, dass** das organische färbende Material einen organischen Lack mit einer der folgenden Bezeichnungen umfasst: D&C Red n°2 Aluminium lake, D&C Red n°3 Aluminium lake, D&C Red n°4 Aluminium lake, D&C Red n°6 Aluminium lake, D&C Red n°6 Barium lake, D&C Red n° 6 Barium/Strontium lake, D&C Red n° 6 Strontium lake, D&C Red n°6 Potassium lake, D&C Red N°7 Aluminium lake, D&C Red n° 7 Barium lake, D&C Red N°7 Calcium lake, D&C Red n° 7 Calcium/Strontium lake, D&C Red n° 7 Zirconium lake, D&C Red n° 8 Sodium lake, D&C Red n°9 Aluminium lake, D&C Red n°9 Barium lake, D&C Red n° 9 Barium/Strontium lake, D&C Red n°9 Zirconium lake, D&C Red n°10 Sodium lake, D&C Red n° 19 Aluminium lake, D&C Red n°19 Barium lake, D&C Red n° 19 Zirconium lake, D&C Red n° 21 Aluminium lake, D&C n° 21 Zirconium lake, D&C n° 22 Aluminium lake, D&C Red n° 27 Aluminium lake, D&C Red n° 27 Aluminium/Titanium/Zirconium lake, D&C Red n° 27 Barium lake, D&C Red n° 27 Calcium lake, D&C Red n°27 Zirconium lake, D&C Red n° 28 Aluminium lake, D&C Red n° 30 lake, D&C Red n° 31 Calcium lake, D&C Red n°33 Aluminium lake, D&C Red n°34 Calcium lake, D&C Red n°36 lake, D&C Red n° 40 Aluminium lake, D&C Blue n°1 Aluminium lake, D&C Green n° 3 Aluminium lake, D&C Orange n°4 Aluminium lake, D&C Orange n°5 Aluminium lake, D&C Orange n° 5 Zirconium lake, D&C Orange n°10 Aluminium lake, D&C Orange n°17 Barium lake, D&C Yellow n° 5 Aluminium lake, D&C Yellow n° 5 Zirconium lake, D&C Yellow n°6 Aluminium lake, D&C Yellow n°7 Zirconium lake, D&C Yellow n°10 Aluminium lake, FD&C Blue n° 1 Aluminium lake, FD&C Red n° 4 Aluminium lake, FD&C Red n°40 Aluminium lake, FD&C Yellow n° 5 Aluminium lake, FD&C Yellow n°6 Aluminium lake.

34. Zusammensetzung nach einem der vorhergehenden Ansprüche, **dadurch gekennzeichnet, dass** das Verbundpigment mindestens ein Bindemittel umfasst, das der Fixierung des organischen färbenden Materials auf dem anorganischen Kern beiträgt.

35. Zusammensetzung nach Anspruch 34, **dadurch gekennzeichnet, dass** das Bindemittel anorganisch ist.

36. Zusammensetzung nach dem vorhergehenden Anspruch, **dadurch gekennzeichnet, dass** das Bindemittel wenigstens einen von einer Silikonverbindung, einer Polymerverbindung, einer Oligomerverbindung, u.ä. umfasst, die wenigstens einen von einem Organosilan, einem Fluoraklorganosilan, einem Polysiloxan, einem Koppelmittel und einer ihrer Mischungen umfasst.

37. Zusammensetzung nach dem vorhergehenden Anspruch, **dadurch gekennzeichnet, dass** das Koppelmittel auf Silan, Titanat, Aluminat, und/oder Zirkonat, oder einer ihrer Mischungen basiert.

38. Zusammensetzung nach Anspruch 36, **dadurch gekennzeichnet, dass** das Bindemittel wenigstens eine Silikonverbindung umfasst.

39. Zusammensetzung nach dem vorhergehenden Anspruch, **dadurch gekennzeichnet, dass** das Bindemittel Polymethylhydrogensiloxan umfasst.

40. Zusammensetzung nach einem der vorhergehenden Ansprüche, **dadurch gekennzeichnet, dass** sie keine nicht umhüllte Titandioxydpartikeln umfasst.

41. Zusammensetzung nach einem der vorhergehenden Ansprüche, **dadurch gekennzeichnet, dass** der zweite Farbstoff eine mehrschichtige Struktur aufweist.

42. Zusammensetzung nach dem vorhergehenden Anspruch, **dadurch gekennzeichnet, dass** der zweite Farbstoff ein Substrat umfasst, das wenigstens einen von einem Metall, einem Metalloxyd und einem Polymer umfasst.

43. Zusammensetzung nach einem der vorhergehenden Ansprüche, **dadurch gekennzeichnet, dass** der zweite Farbstoff Perlmutt umfasst.

44. Zusammensetzung nach dem vorhergehenden Anspruch, **dadurch gekennzeichnet, dass** der zweite Farbstoff wenigstens einen von einem Glimmer, einem künstlichen Glimmer, einem Talk, einem Siliziumoxyd und einem Aluminiumoxyd umfasst.

45. Zusammensetzung nach einem der vorhergehenden Ansprüche, **dadurch gekennzeichnet, dass** der zweite Farbstoff Glaspartikeln umfasst.

46. Zusammensetzung nach einem der vorhergehenden Ansprüche, **dadurch gekennzeichnet, dass** der zweite Farbstoff wenigstens einen ganiochromatischen Farbstoff umfasst.

47. Zusammensetzung nach einem der vorhergehenden Ansprüche, **dadurch gekennzeichnet, dass** der zweite Farbstoff reflektierende Partikel umfasst.

48. Zusammensetzung nach einem der vorhergehenden Ansprüche, **dadurch gekennzeichnet, dass** der zweite Farbstoff an der Oberfläche metallisierte Partikel umfasst.

49. Zusammensetzung nach Anspruch 47, **dadurch gekennzeichnet, dass** die reflektierenden Partikel Partikel mit einschließen, die ein künstliches Substrat umfassen, das wenigstens teilweise durch wenigstens eine Schicht wenigstens eines Metalls oder einer Metallverbindung beschichtet ist.

50. Zusammensetzung nach einem der vorhergehenden Ansprüche, **dadurch gekennzeichnet, dass** sie eine feste Form aufweist.

51. Zusammensetzung nach einem der vorhergehenden Ansprüche, **dadurch gekennzeichnet, dass** sie eine flüssige, pastöse oder gelierte Form aufweist.

52. Zusammensetzung nach einem der Ansprüche 1 bis 51, **dadurch gekennzeichnet, dass** es sich hierbei um einen Lippenstift handelt.

53. Zusammensetzung nach einem der Ansprüche 1 bis 51, **dadurch gekennzeichnet, dass** es sich hierbei um eine Foundation handelt

54. Zusammensetzung nach einem der Ansprüche 1 bis 51, **dadurch gekennzeichnet, dass** es sich hierbei um einen Nagellack handelt.

55. Zusammensetzung nach einem der Ansprüche 1 bis 51, **dadurch gekennzeichnet, dass** es sich hierbei um Wimperntusche handelt.

56. Zusammensetzung nach einem der Ansprüche 1 bis 51, **dadurch gekennzeichnet, dass** es sich hierbei um ein Produkt zur Färbung von Haarfasern handelt.

## Claims

1. A composition for application to the skin, the lips, the nails, and/or hair, said composition including:
· at least one first coloring agent, ranging from 0.1% to 20% by weight relative to the total composition weight, in an amount sufficient to color the composition, comprising particles of at least one non-interference composite pigment, said particles comprising:
· an inorganic core; and
· an at least partial coating of at least one organic coloring substance;
· the composition further including:
· at least one second coloring agent, ranging from 0.1% to 50% by weight relative to the total composition weight, in an amount sufficient to produce a specific optical effect in the composition which is perceptible to the naked eye;
· with the second coloring agent comprising reflective particles, nacres and/or a goniochromatic coloring agent.

2. A composition according to claim 1, **characterized by** the fact that the specific optical effect is a point highlight effect.

3. A composition according to claim 1, **characterized by** the fact that the specific optical effect is a goniochromatic effect.

4. A composition according to claim 1, **characterized by** the fact that the specific optical effect is a speckled effect.

5. A composition according to any preceding claim, **characterized by** the fact that the first coloring agent is present in the composition in an amount ranging from 0.1% to 10% by weight relative to the total composition weight.

6. A composition according to the preceding claim, **characterized by** the fact that the first coloring agent is present in the composition in an amount ranging from 0.5% to 5% by weight relative to the total composition weight.

7. A composition according to any preceding claim, **characterized by** the fact that the second coloring agent is present in the composition in an amount ranging from 0.5% to 40% by weight relative to the total composition weight.

8. A composition according to the preceding claim, **characterized by** the fact that the second coloring agent is present in the composition in an amount ranging from 1% to 20% by weight relative to the total composition weight.

9. A composition according to any preceding claim, **characterized by** the fact that the weight ratio of the second coloring agent to the first coloring agent ranges from 0.1 to 50.

10. A composition according to the preceding claim, **characterized by** the fact that the weight ratio of the second coloring agent to the first coloring agent ranges from 0.5 to 30.

11. A composition according to the preceding claim, **characterized by** the fact that the weight ratio of the second coloring agent to the first coloring agent ranges from 1 to 10.

12. A composition according to any preceding claim, **characterized by** the fact that the organic coloring substance comprises at least one organic pigment.

13. A composition according to any preceding claim, **characterized by** the fact that the organic coloring substance comprises at least one organic lake.

14. A composition according to any preceding claim, **characterized by** the fact that the mean size of the inorganic core ranges from 1 nm to 100 nm.

15. A composition according to the preceding claim, **characterized by** the fact that the mean size of the inorganic core ranges from 5 nm to 75 nm.

16. A composition according to the preceding claim, **characterized by** the fact that the mean size of the inorganic core ranges from 10 nm to 50 nm.

17. A composition according to any preceding claim, **characterized by** the fact that the specific surface area of the inorganic core ranges from 1, m²/g to 1000 m²/g.

18. A composition according to the preceding claim, **characterized by** the fact that the specific surface area of the inorganic core ranges from 10 m²/g to 600 m²/g.

19. A composition according to the preceding claim, **characterized by** the fact that the specific surface area of the inorganic core ranges from 20 m²/g to 400 m²/g.

20. A composition according to any preceding claim, **characterized by** the fact that the inorganic core comprises a material chosen from a metallic salt, a metal oxide, an alumina, a glass, a ceramic, a graphite, a silica, a silicate, a synthetic mica, and a mixture thereof.

21. A composition according to the preceding claim, **characterized by** the fact that the inorganic core comprises a metal oxide chosen from an oxide of titanium, iron, cerium, zirconium, zinc, aluminum, iron blue, or chromium.

22. A composition according to the preceding claim, **characterized by** the fact that the inorganic core comprises a metal oxide chosen from an oxide of titanium, iron, cerium, zirconium, zinc, or aluminum.

23. A composition according to the preceding claim, **characterized by** the fact that the inorganic core comprises titanium dioxide.

24. A composition according to claim 20, **characterized by** the fact that the inorganic core comprises at least one silicate chosen from an aluminosilicate and a borosilicate.

25. A composition according to claim 20, **characterized by** the fact that the inorganic core comprises silica.

26. A composition according to any preceding claim, **characterized by** the fact that the proportion by weight of the organic coloring substance ranges from 10 to 500 parts by weight per 100 parts by weight of inorganic core.

27. A composition according to the preceding claim, **characterized by** the fact that the proportion by weight of the organic coloring substance ranges from 20 to 250 parts by weight per 100 parts by weight of inorganic core.

28. A composition according to the preceding claim, **characterized by** the fact that the proportion by weight of the organic coloring substance ranges from 40 to 125 parts by weight per 100 parts by weight of inorganic core.

29. A composition according to any preceding claim, **characterized by** the fact that the organic coloring substance is selected from carmine cochineal, an organic pigment of an azo, anthraquinone, indigo, xanthene, pyrene, quinoline, triphenylmethane or fluorane dye, an organic lake, an insoluble sodium, potassium, calcium, barium, aluminum, zirconium, strontium or titanium salt, and an acid dye.

30. A composition according to the preceding claim, **characterized by** the fact that the acid dye is selected from an azo, anthraquinone, indigo, xanthene, pyrene, quinoline, triphenylmethane and fluorane dye, and any other dye including at least one carboxylic or sulfonic acid group.

31. A composition according to any preceding claim, **characterized by** the fact that the organic coloring substance comprises an organic lake supported by an organic support comprising at least one colophane or aluminum benzoate.

32. A composition according to any preceding claim, **characterized by** the fact that the organic coloring substance comprises an organic pigment having one of the following denominations: D&C Blue No.4, D&C Brown No.1, D&C Green No.5, D&C Green No.6, D&C Orange No.4, D&C Orange No.5, D&C Orange No.10, D&C Orange No.11, D&C Red No.6, D&C Red No.7, D&C Red No.17, D&C Red No.21, D&C Red No.22, D&C Red No.27, D&C Red No.28, D&C Red No.30, D&C Red No.31, D&C Red No.33, D&C Red No.34, D&C Red No.36, D&C Violet No.2, D&C Yellow No.7, D&C Yellow No.8, D&C Yellow No.10, D&C Yellow No.11, FD&C Blue No.1, FD&C Green No.3, FD&C Red No.40, FD&C Yellow No.5, and FD&C Yellow No.6.

33. A composition according to any preceding claim, **characterized by** the fact that the organic coloring substance comprises an organic lake having one of the following denominations: D&C Red No.2 Aluminum lake, D&C Red No.3 Aluminum lake, D&C Red No.4 Aluminum lake, D&C Red No.6 Aluminum lake, D&C Red No.6 Barium lake, D&C Red No.6 Barium/Strontium lake, D&C Red No.6 Strontium lake, D&C Red No.6 Potassium lake, D&C Red No.7 Aluminum lake, D&C Red No.7 Barium lake, D&C Red No.7 Calcium lake, D&C Red No.7 Calcium/Strontium lake, D&C Red No.7 Zirconium lake, D&C Red No.8 Sodium lake, D&C Red No.9 Aluminum lake, D&C Red No.9 Barium lake, D&C Red No.9 Barium/Strontium lake, D&C Red No.9 Zirconium lake, D&C Red No.10 Sodium lake, D&C Red No.19 Aluminum lake, D&C Red No.19 Barium lake, D&C Red No.19 Zirconium lake, D&C Red No.21 Aluminum lake, D&C Red No.21 Zirconium lake, D&C Red No.22 Aluminum lake, D&C Red No.27 Aluminum lake, D&C Red No.27 Aluminum/Titanium/ Zirconium lake, D&C Red No.27 Barium lake, D&C Red No.27 Calcium lake, D&C Red No.27 Zirconium lake, D&C Red No.28 Aluminum lake, D&C Red No.30 lake, D&C Red No.31 Calcium lake, D&C Red No.33 Aluminum lake, D&C Red No.34 Calcium lake, D&C Red No.36 lake, D&C Red No.40 Aluminum lake, D&C Blue No.1 Aluminum lake, D&C Green No.3 Aluminum lake, D&C Orange No.4 Aluminum lake, D&C Orange No.5 Aluminum lake, D&C Orange No.5 Zirconium lake, D&C Orange No.10 Aluminum lake, D&C Orange No.17 Barium lake, D&C Yellow No.5 Aluminum lake, D&C Yellow No.5 Zirconium lake, D&C Yellow No.6 Aluminum lake, D&C Yellow No.7 Zirconium lake, D&C Yellow No.10 Aluminum lake, FD&C Blue No.1 Aluminum lake, FD&C Red No.4 Aluminum lake, FD&C Red No.40 Aluminum lake, FD&C Yellow No.5 Aluminum lake, and FD&C Yellow No.6 Aluminum lake.

34. A composition according to any preceding claim, **characterized by** the fact that the composite pigment comprises at least one binder contributing to fixing the organic coloring substance on the inorganic core.

35. A composition according to claim 34, **characterized by** the fact that the binder is inorganic.

36. A composition according to the preceding claim, **characterized by** the fact that the binder comprises at least one silicone compound, a polymeric compound, an oligomeric compound or the like comprising at least an organosilane, an organosilane of a fluoroalkylated organosilane, a polysiloxane or a coupling agent, and a mixture thereof.

37. A composition according to the preceding claim, **characterized by** the fact that the coupling agent is based on a silane, a titanate, an aluminate and/or a zirconate, or a mixture thereof.

38. A composition according to claim 36, **characterized by** the fact that the binder comprises at least one silicone compound.

39. A composition according to the preceding claim, **characterized by** the fact that the binder comprises polymethylhydrogen siloxane.

40. A composition according to any preceding claim, **characterized by** the fact that it is free of uncoated particles of titanium dioxide.

41. A composition according to any preceding claim, **characterized by** the fact that the second coloring agent has a multilayered structure.

42. A composition according to the preceding claim, **characterized by** the fact that the second coloring agent comprises a substrate comprising at least one metal, a metal oxide and a polymer.

43. A composition according to any preceding claim, **characterized by** the fact that the second coloring agent comprises nacres.

44. A composition according to the preceding claim, **characterized by** the fact that the second coloring agent comprises at least one mica, a synthetic mica, a talc, a silica, or an alumina.

45. A composition according to any preceding claim, **characterized by** the fact that the second coloring agent comprises particles based on glass.

46. A composition according to any preceding claim, **characterized by** the fact that the second coloring agent comprises at least one goniochromatic coloring agent.

47. A composition according to any preceding claim, **characterized by** the fact that the second coloring agent comprises reflective particles.

48. A composition according to any preceding claim, **characterized by** the fact that the second coloring agent comprises particles which are surface metallized.

49. A composition according to claim 47, **characterized by** the fact that the reflective particles comprise particles comprising a synthetic substrate at least partially coated with at least one layer of at least one metal or metallic compound.

50. A composition according to any preceding claim, **characterized by** the fact that it is in solid form.

51. A composition according to any preceding claim, **characterized by** the fact that it is in liquid, paste, or gel form.

52. A composition according to any one of claims 1 to 51, **characterized in that** it is a lipstick.

53. A composition according to any one of claims 1 to 51, **characterized in that** it is a foundation.

54. A composition according to any one of claims 1 to 51, **characterized in that** it is a nail polish.

55. A composition according to any one of claims 1 to 51, **characterized in that** it is a mascara.

56. A composition according to any one of claims 1 to 51, **characterized in that** it is a product for coloring hair fibers.
